# EUROPÄISCHE PATENTSCHRIFT

(11) **EP 1 874 769 B1**
(45) Veröffentlichungstag und Bekanntmachung des Hinweises auf die Patenterteilung: **14.09.2011**
(21) Anmeldenummer: 06723838.6
(22) Anmeldetag: 30.03.2006
(51) Int. Cl.: C07D 471/04, A61K 31/435, A61P 35/00

(54) **NEUARTIGE AZA-HETEROZYKLEN ALS KINASE-INHIBITOREN**
NOVEL AZA- HETEROCYCLES SERVING AS KINASE INHIBITORS
NOUVEAUX AZA-HETEROCYCLES CONSTITUANT DES INHIBITEURS DE KINASES

(30) Priorität: 25.04.2005 DE 102005019094; 19.01.2006 DE 102006002649
(43) Veröffentlichungstag der Anmeldung: 09.01.2008
(73) Patentinhaber: Merck Patent GmbH, 64293 Darmstadt (DE)
(72) Erfinder: HEINRICH, Timo, 64823 Gross-Umstadt (DE); BLAUKAT, Andree, 64367 Muehltal (DE); STAEHLE, Wolfgang, 55218 Ingelheim (DE); GREINER, Hartmut, 64331 Weiterstadt (DE); KORDOWICZ, Maria, 64347 Griesheim (DE)
(86) Internationale Anmeldenummer: PCT/EP2006/002871
(87) Internationale Veröffentlichungsnummer: WO 2006/114180

(56) Entgegenhaltungen:
- WO-A-2004/016609
- WO-A-2004/099205
- WO-A-2005/116028

## Beschreibung

Die Erfindung betrifft Verbindungen der Formel I, 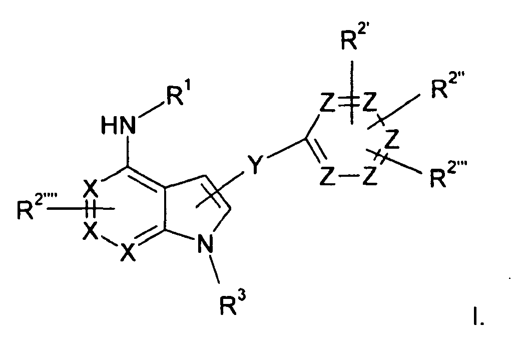 worin
- R¹: H, A, Ar, Ar-A oder A-Ar,
- A: unverzweigtes, verzweigtes oder cyclisches Alkyl mit 1-14 C- Atomen, worin eine oder zwei CH₂-Gruppen durch ein O- o- der S-Atom und/oder durch eine NH, NA, CONH, NHCO oder -CH=CH- Gruppe und/oder auch 1-7 H-Atome durch Hal er- setzt sein können, und worin eine oder zwei CH₃-Gruppen durch NH₂, NAH, NA₂, NHCOOA, NHCONHA, NHCONHAr oder CN ersetzt sein können,
- Ar: einen ein- oder zweikernigen aromatischen Homo- oder He- terocyclus mit 1 bis 4 N-, O- und/oder S-Atomen und 5 bis 10 Gerüstatomen, der unsubstituiert oder ein-, zwei- oder drei- fach durch Carbonylsauerstoff, Hal, A, OH, OA, NH₂, NHA, NA₂, NO₂, CN, OCN, SCN, COOH, COOA, CONH₂, CONHA, CONA₂, NHCOA, NHCONH₂, NHSO₂A, CHO, COA, SO₂NH₂ und/oder S(O)_{g}A substituiert sein kann,
- Ar-A: Aryl-alkyl
- A-Ar: Alkyl-aryl
- Hal: F, Cl, Br oder I,
- X: CH oder N, wobei in jeder Verbindung der Formel I eine Gruppierung X N und zwei Gruppierungen X CH sind,
- Y: CH₂ oder eine gesättigte Bindung,
- Z: CH oder N, wobei in jeder Verbindung der Formel I höchs- tens zwei Gruppierungen Z N und vorzugsweise eine oder keine Gruppierung Z N sind,
- R², R^{2"}, R^{2"'}, R^{2""}: jeweils unabhängig voneinander H, Hal, OH, CN, NH₂, unverzweigtes oder verzweigtes Alkyl mit 1-4, 5 oder 6 C- Atomen, worin eine CH₂-Gruppe durch ein O oder S-Atom und/oder durch eine NH, NA, CONH oder -CH=CH- Gruppe und/oder auch 1-4 H-Atome durch Hal ersetzt sein können, und worin eine CH₃-Gruppe durch NH₂, NAH, NA₂, CN oder Ar ersetzt sein kann,
- R³: H, A oder Ar-A,
- g: 0, 1 oder 2 und
- -̅ -̅ -̅ -̅ -̅: eine Einfach- oder Doppelbindung
bedeuten,
3-(Aminophenyl)-1H-pyrrolo[3,2-c]pyridin-4-amin nicht umfasst ist, sowie ihre pharmazeutisch unbedenklichen Salze, Solvate und Stereoisomere, einschließlich deren Mischungen in allen Verhältnissen.

Es wurde gefunden, dass die Verbindungen der Formel I die Signaltransduktion, die durch Kinasen vermittelt wird, insbesondere durch Tyrosinkinasen, hemmen, regulieren und/oder modulieren können. Insbesondere eignen sich die erfindungsgemäßen Verbindungen als Inhibitoren von Tyrosinkinasen. So können erfindungsgemäße Medikamente und pharmazeutische Zusammensetzungen wirksam zur Behandlung von Krankheiten eingesetzt werden, die durch Kinasen und/oder durch kinase-vermittelte Signaltransduktion verursacht, vermittelt und/oder propagiert werden. Somit eignen sich die erfindungsgemäßen Verbindungen zur Behandlung und Prophylaxe von Krebs, Tumorwachstum, Arteriosklerose, diabetischer Retinopathie, Entzündungserkrankungen, Psoriasis und dergleichen bei Säugetieren.

### Hintergrund der Erfindung

Krebs ist eine Krankheit, deren Ursachen unter anderem in einer gestörten Signaltransduktion zu sehen sind. Insbesondere deregulierte Signaltransduktion über Tyrosinkinasen spielt eine zentrale Rolle beim Wachstum und der Ausbreitung von Krebs (Blume-Jensen, P. und T. Hunter, Nature 411: 355-365, 2001; Hanahan D. und R. A. Weinberg, Cell 100:57-70, 2000). Tyrosinkinasen und insbesondere Rezeptor-Tyrosinkinasen sowie die an sie bindenden Wachstumsfaktoren können so an deregulierter Apoptose, Gewebeinvasion, Metastasierung und allgemein an Signaltransduktionsmechanismen, die zu Krebs führen, beteiligt sein.

Wie bereits erwähnt, ist einer der Hauptmechanismen, durch den die Zellregulation bewirkt wird, die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein sehr weit verbreiteter Prozess in Zellen ist, und da Zellphänotypen größtenteils von der Aktivität dieser Wege beeinflusst werden, wird zur Zeit angenommen, dass eine große Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (siehe Übersichtsartikel: Weinstein-Oppenheimer et al., Pharma. &. Therap. 88:229-279, 2000). Verschiedene Möglichkeiten zur Hemmung, Regulation und Modulation von Kinasen umfassen beispielsweise die Bereitstellung von Antikörpern, antisense-Ribozymen und Inhibitoren. In der Onkologieforschung sind insbesondere Tyrosinkinasen vielversprechende Targets. So sind zahlreiche synthetische kleine Moleküle als Tyrosinkinase-Inhibitoren zur Behandlung von Krebs in der klinischen Entwicklung z.B. Iressa^{®} oder Gleevec^{®}. Allerdings sind hier noch zahlreiche Probleme zu lösen, wie Nebenwirkungen, Dosierung, Resistenz des Tumors, Tumorspezifität und Patientenauswahl.

Bei den Tyrosinkinasen handelt es sich um eine Klasse von Enzymen, die die Übertragung des endständigen Phosphats des Adenosintriphosphats auf Tyrosinreste bei Proteinsubstraten katalysieren. Man nimmt an, dass den Tyrosinkinasen bei verschiedenen Zellfunktionen über die Substratphosphorylierung eine wesentliche Rolle bei der Signaltransduktion zukommt. Obwohl die genauen Mechanismen der Signaltransduktion noch unklar sind, wurde gezeigt, dass die Tyrosinkinasen wichtige Faktoren bei der Zellproliferation, der Karzinogenese und der Zelldifferenzierung darstellen.
Die Tyrosinkinasen lassen sich in Rezeptor-Tyrosinkinasen und zytosolische Tyrosinkinasen einteilen. Die Rezeptor-Tyrosinkinasen weisen einen extrazellulären Teil, einen Transmembranteil und einen intrazellulären Teil auf, während die zytosolischen Tyrosinkinasen ausschließlich intrazellulär vorliegen.

Die Rezeptor-Tyrosinkinasen bestehen aus einer Vielzahl von Transmembranrezeptoren mit unterschiedlicher biologischer Wirksamkeit. So wurden ungefähr 20 verschiedene Unterfamilien von Rezeptor-Tyrosinkinasen identifiziert. Eine Tyrosinkinase-Unterfamilie, die die Bezeichnung EGFR- oder HER-Unterfamilie trägt, besteht aus EGFR, HER2, HER3 und HER4. Zu den Liganden dieser Rezeptor-Unterfamilie zählen der Epithel-Wachstumsfaktor (EGF), der Gewebewachstumsfaktor (TGF-α), Amphiregulin, HB-EGF, Betacellulin und Heregulin. Die Insulin-Unterfamilie, zu der INS-R, IGF-IR und IR-R zählen, stellt eine weitere Unterfamilie dieser Rezeptor-Tyrosinkinasen dar. Die PDGF-Unterfamilie beinhaltet den PDGF-α- and -β-Rezeptor, CSFIR, c-kit und FLK-II. Außerdem gibt es die FLK-Familie, die aus dem Kinaseinsertdomänenrezeptor (KDR) oder VEGFR-2, der fötalen Leberkinase-1 (FLK-1), der fötalen Leberkinase-4 (FLK-4) und der fms-Tyrosinkinase-1 (flt-1) oder VEGFR-1 besteht. Die PDGF- und FLK-Familie werden üblicherweise aufgrund der zwischen den beiden Gruppen bestehenden Ähnlichkeiten in der Gruppe der Splitkinase-Domänen Rezeptor-Tyrosinkinasen zusammengefasst (Laird, A. D. und J. M. Cherrington, Expert. Opin. Investig. Drugs 12(1):51-64, 2003). Für eine genaue Diskussion der Rezeptor-Tyrosinkinasen siehe die Arbeit von Plowman et al., DN & P 7(6):334-339, 1994).

Die zytosolischen Tyrosinkinasen bestehen ebenfalls aus einer Vielzahl von Unterfamilien, darunter Src, Frk, Btk, Csk, Abl, Zap70, Fes/Fps, Fak, Jak, Ack, and LIMK. Jede dieser Unterfamilien ist weiter in verschiedene Untergruppen unterteilt. So stellt zum Beispiel die Src-Unterfamilie eine der größten Unterfamilien dar. Sie beinhaltet Src, Yes, Fyn, Lyn, Lck, Blk, Hck, Fgr und Yrk. Die Src-Enzymunterfamilie wurde mit der Onkogenese in Verbindung gebracht. Für eine genauere Diskussion der zytosolischen Tyrosinkinasen, siehe die Arbeit von Bolen, Oncogene, 8:2025-2031, 1993.
Sowohl die Rezeptor-Tyrosinkinasen als auch die zytosolischen Tyrosinkinasen sind an Signalübertragungswegen der Zelle, die zu Leidenszuständen wie Krebs, Schuppenflechte und Hyperimmunreaktionen führen, beteiligt.

Die vorliegende Erfindung betrifft nun Verbindungen der Formel I, vorzugsweise als Regulatoren, Modulatoren oder Inhibitoren von Rezeptor-Tyrosinkinasen der Insulin-Unterfamilie, zu der der Insulinrezeptor IR, der "insulin like growth factor-1 receptor" IGF-1R und der "insulin related receptor" IRR zählen. Besondere Wirkung zeigen die erfindungsgemäßen Verbindungen bei der Inhibierung der Rezeptor-Tyrosinkinase IGF-1R.

Wie zuvor erwähnt, gehört der insulinähnliche Wachstumsfaktor-1-Rezeptor (IGF-1 R) zur Familie der transmembranen Tyrosinkinase-Rezeptoren, wie der aus Blutplättchen stammende Wachstumsfaktor-Rezeptor (platelet derived growth factor receptor), der epidermale Wachstumsfaktor-Rezeptor und der Insulinrezeptor. Es gibt zwei bekannte Liganden für den IGF-1R-Rezeptor. Dabei handelt es sich um IGF-1 und IGF-2. Wie hier verwendet, bezieht sich der Begriff "IGF" sowohl auf IGF-1 als auch auf IGF-2. Eine Übersicht über die insulinähnliche Wachstumsfaktor-Familie von Liganden, Rezeptoren und Bindungsproteinen findet sich in Krywicki und Yee, Breast Cancer Research and Treatment, 22:7-19, 1992.

Durch IGF/IGF-1R hervorgerufene Erkrankungen sind durch eine anomale Aktivität oder Hyperaktivität von IGF/IGF-1R gekennzeichnet. Anomale IGF-Aktivität betrifft entweder: (1) IGF- oder IGF-1 R-Expression in Zellen, die gewöhnlich kein IGF oder IGF-1 R exprimieren; (2) erhöhte IGF- oder IGF-1 R-Expression, die zu unerwünschter Zellproliferation, wie Krebs, führt; (3) erhöhte IGF- oder IGF-1 R-Aktivität, die zu unerwünschter Zellproliferation, wie Krebs, und/oder zu Hyperaktivität von IGF oder IGF-1R führt. Hyperaktivität von IGF oder IGF 1 R bezieht sich entweder auf eine Amplifikation des Gens, das IGF-1, IGF-2, IGF-1R codiert, oder die Erzeugung eines Spiegels der IGF-Aktivität, der mit einer Zellproliferationserkrankung korreliert werden kann (d.h. mit steigendem IGF-Spiegel steigt die Schwere eines oder mehrerer Symptome der Zellproliferationserkrankung) die biologische Verfügbarkeit von IGF-1 und IGF-2 kann auch durch das Vorhandensein oder Fehlen eines Satzes von IGF-Bindungsproteinen (IGF-BP's) beeinflusst werden, von denen sechs bekannt sind. Hyperaktivität von IGF/IGF-1 R kann auch durch eine Herunterregulation von IGF-2 verursacht werden, das eine IGF-2-Bindungsdomäne, aber keine intrazelluläre Kinasedomäne enthält. Beispiele für durch IGF/IGF-1 R hervorgerufene Erkrankungen umfassen die verschiedenen mit IGF/IGF-1R in Zusammenhang stehenden malignen Erkrankungen beim Menschen, über die Cullen et al., Cancer Investigation, 9(4):443-454, 1991, eine Übersicht geben. Zur klinischen Bedeutung und der Rolle von IGF/IGF-IR bei der Regulation der Osteoblastenfunktion siehe Schmid, Journal of Internal Medicine, 234:535-542, 1993.

Die Aktivitäten von IGF-1 R umfassen somit: (1) Phosphorylierung von IGF-1 R-Protein; (2) Phosphorylierung eines IGF-1R-Proteinsubstrats; (3) Wechselwirkung mit einem IGF-Adapterprotein; (4) Oberflächenexpression des IGF-1R-Proteins. Weitere Aktivitäten des IGF-1R-Proteins können unter Verwendung von Standardtechniken identifiziert werden. Die IGF-1 R-Aktivität kann durch Messen einer oder mehrerer der folgenden Aktivitäten untersucht werden: (1) Phosphorylierung von IGF-1 R; (2) Phosphorylierung eines IGF-1 R-Substrats; (3) Aktivierung eines IGF-1 R-Adaptermoleküls und (4) Aktivierung stromabwärts gelegener Signalmoleküle und/oder (5) gesteigerte Zellteilung. Diese Aktivitäten können unter Verwendung nachstehend beschriebener und im Stand der Technik bekannter Techniken gemessen werden.

IGF-1 R wurde als essentiell für die Erzeugung und Aufrechterhaltung des transformierten Phänotyps in mehreren Zelltypen in vitro und in vivo angesehen (R. Baserga, Cancer Research 55:249- 252, 1995). Von Herbimycin A wurde gesagt, dass es die IGF-1R-Protein-Tyrosinkinase und die Zellproliferation in menschlichen Brustkrebszellen hemmt (Sepp-Lorenzino et al., J. Cell Biochem. Suppl. 18b:246, 1994). Experimente zur Untersuchung der Rolle von IGF-1 R bei der Transformation, bei denen Antisense-Strategien, dominant negative Mutationen und Antikörper gegen IGF-1R verwendet wurden, führten zu der Hypothese, dass IGR-1 R ein bevorzugtes Ziel für therapeutische Eingriffe sein kann.

Über seine Rolle bei der Nährstoffzufuhr und bei Diabetes Typ II hinaus, wurde IGF-1 R zudem mit mehreren Krebsarten in Verbindung gebracht. Beispielsweise hat man IGF-1 als autokrinen Wachstumsstimulator bei mehreren Tumorarten, z.B. menschlichen Brustkrebskarzinomzellen (Arteago et al., J. Clin. Invest., 84:1418-1423, 1989) und kleinen Lungentumorzellen (Macauley et al., Cancer Res., 50:2511-2517, 1989) identifiziert. Außerdem scheint IGF-1, der untrennbar mit dem normalen Wachstum und der normalen Differenzierung des Nervensystems verknüpft ist, auch ein autokriner Stimulator menschlicher Gliome zu sein. Sandberg-Nordqvist et al., Cancer Res., 53:2475-2478, 1993.

Ein Beispiel für die potenzielle Beteiligung von IGF-2 an Kolorektalkrebs könnte die Heraufregulation der IGF-2-mRNA in Kolontumoren verglichen mit normalem Dickdarmgewebe sein. (Zhang et al., Science 276:1268-1272, 1997) IGF-2 kann auch eine Rolle bei der durch Hypoxie verursachten Neuvaskularisation von Tumoren spielen. (Mines et al., Int. J. Mol. Med. 5:253-259, 2000) IGF-2 kann auch über die Aktivierung einer Insulinrezeptor-Isoform-A eine Rolle bei der Tumorigenese spielen. Die IGF-2-Aktivierung der Insulinrezeptor-Isoform-A aktiviert Signalwege für das Überleben von Zellen, aber das Ausmaß ihres Beitrags zu Tumorzellwachstum und -überleben ist zurzeit noch unbekannt. Die Kinasedomäne der Insulinrezeptor-Isoform-A ist mit derjenigen des Standard-Insulinrezeptors identisch (Scalia et al., J. Cell Biochem. 82:610-618, 2001).

Die Bedeutung von IGF-1 R und seiner Liganden in Zelltypen in Kultur (Fibroblasten, Epithelzellen, glatte Muskelzellen, T-Lymphozyten, Myeloidzellen, Chondrozyten und Osteoblasten (die Stammzellen des Knochenmarks)) wird durch die Fähigkeit von IGF-1, Zellwachstum und - proliferation zu stimulieren, demonstriert (Goldring und Goldring, Eukaryotic Gene Expression, 1:301-326, 1991). In einer Reihe neuerer Veröffentlichungen legen Baserga et al. nahe, dass IGF-1R eine zentrale Rolle beim Mechanismus der Transformation spielt und als solcher ein bevorzugtes Ziel für therapeutische Eingriffe bei einem breiten Spektrum menschlicher maligner Erkrankungen sein könnte (Baserga, Cancer Res., 55:249-252, 1995; Baserga, Cell, 79:927-930, 1994; Coppola et al., Mol. Cell. Biol., 14:4588-4595, 1994; Baserga, Trends in Biotechnology, 14:150-152, 1996; H.M. Khandwala et al., Endocrine Reviews, 21:215-244, 2000).

Die wichtigsten Krebsarten, die unter Verwendung einer erfindungsgemäßen Verbindung behandelt werden können, umfassen Brustkrebs, Prostatakrebs, Kolorektalkrebs, kleinzelligen Lungenkrebs, nicht-kleinzelligen Lungenkrebs, das multiple Myelom sowie das Nierenzellkarzinom und das Endometriumkarzinom.

IGF-1 wurde auch mit der Neovaskularisation der Retina in Verbindung gebracht. Bei einigen Patienten mit hohen IGF-1-Spiegeln wurde eine proliferative Diabetes-Retinopathie beobachtet. (L.E. Smith et al., Nature Medicine, 5:1390-1395, 1999)

Die erfindungsgemäßen Verbindungen können sich aber auch als Mittel zur Alterungsverzögerung eignen. Es wurde beobachtet, dass es eine Verbindung zwischen IGF-Signalen und Alterung gibt. Experimente haben gezeigt, dass Säuger mit kalorienreduzierter Diät niedrige Insulin- und IGF-1-Spiegel aufweisen und eine längere Lebensdauer aufweisen. Ähnliche Beobachtungen wurden auch bei Insekten gemacht (siehe C. Kenyon, Cell, 105:165-168, 2001; E. Strauss, Science, 292:41-43, 2001; K.D. Kimura et al., Science, 277:942-946, 1997; M. Tatar et al., Science, 292:107-110, 2001).

Gegenstand der vorliegenden Erfindung ist somit auch die Verwendung der Verbindungen der Formel I zur Vorbeugung und/oder Behandlung von Erkrankungen im Zusammenhang mit unregulierter oder gestörter Rezeptor-Aktivität. Insbesondere lassen sich die erfindungsgemäßen Verbindungen deshalb bei der Behandlung gewisser Krebsformen einsetzen, wie bspw. Brustkrebs, Prostatakrebs, Darmkrebs, kleinzelliger und nichtkleinzelliger Lungenkrebs, multiples Myelom, Nierenzellkarzinom oder Korpuskarzinom.

Weiterhin denkbar ist die Verwendung der erfindungsgemäßen Verbindungen zur Behandlung von diabetischer Retinopathie oder zur Verzögerung des Alterungsprozesses. Insbesondere eignen sie sich zur Verwendung in diagnostischen Verfahren zu Erkrankungen im Zusammenhang mit unregulierter oder gestörter IGF-1R-Aktivität.

Zudem können die erfindungsgemäßen Verbindungen verwendet werden, um bei gewissen existierenden Krebs-Chemotherapien und - Bestrahlungen additive oder synergistische Effekte zu erzielen und/oder, um die Wirksamkeit gewisser existierender Krebs-Chemotherapien und - Bestrahlungen wiederherzustellen.

Eine Reihe von aza-Indol-Verbindungen wurden bisher als Kinase-Inhibitoren beschrieben, etwa in der WO 02/092603, WO 04/043388 oder der WO 04/016609.
WO 2004/099205 A1 beschreibt Azaindolverbindungen, die in 5-Stellung einen Säureamidrest aufweisen.
WO 2005/116028 A2 offenbart weitere Azaindolverbindungen und deren Verwendung zur Krebsbehandlung.

Der Erfindung lag nunmehr die Aufgabe zugrunde, neue Verbindungen mit vorteilhaften therapeutischen Eigenschaften aufzufinden, die zur Herstellung von Arzneimitteln verwendet werden können.
So ist die Identifikation und Bereitstellung von chemischen Verbindungen, die die Signaltransduktion der Tyrosinkinasen spezifisch hemmen, regulieren und/oder modulieren, wünschenswert und daher ein Ziel der vorliegenden Erfindung.

### Beschreibung der Erfindung

Es wurde gefunden, dass die Verbindungen der Formel I und ihre Salze bei guter Verträglichkeit sehr wertvolle pharmakologische Eigenschaften besitzen. Insbesondere wurde gefunden, dass die erfindungsgegenständlichen Verbindungen der Formel I überraschenderweise wirksame Kinase-Inhibitoren darstellen, wobei sie insbesondere eine Tyrosinkinaseninhibierende Wirkung, und in besonderem Maße eine IGF-R1 inhibierende Wirkung zeigen.

Generell gilt, dass sämtliche Reste, die mehrfach auftreten, gleich oder verschieden sein können, d.h. unabhängig voneinander sind. Vor- und nachstehend haben die Reste bzw. Parameter die für die Formel I angegebenen Bedeutungen, falls nicht ausdrücklich etwas anderes angegeben ist.
Dementsprechend sind Gegenstand der Erfindung insbesondere diejenigen Verbindungen der Formel I, in denen mindestens einer der genannten Reste eine der nachstehend angegebenen bevorzugten Bedeutungen hat.

Hal bedeutet Fluor, Chlor, Brom oder Iod, insbesondere Fluor oder Chlor.

A bedeutet Alkyl, ist unverzweigt (linear), verzweigt oder cyclisch, und hat 1, 2, 3, 4, 5, 6, 7, 8, 9, 10, 11, 12, 13 oder 14 C-Atome.

So bedeutet A beispielsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, lineares oder verzweigtes Heptyl, Octyl, Nonyl oder Decyl.

A bedeutet bevorzugt Alkyl mit 1, 2, 3, 4, 5 oder 6 C-Atomen, worin eine oder zwei CH₂-Gruppen durch O- oder S-Atome und/oder durch NH, NA, CONH, NHCO oder -CH=CH-Gruppen und/oder auch 1-7 H-Atome durch F und/oder Cl ersetzt sein können, wie z.B. Methyl, Ethyl, Propyl, Isopropyl, Butyl, Isobutyl, sek.-Butyl, tert.-Butyl, Pentyl, Hexyl, Trifluormethyl, Pentafluorethyl, 1,1-Difluormethyl, 1,1,1-Trifluorethyl, Methoxy, Ethoxy, n-Propoxy, Isopropoxy, n-Butoxy, Isobutoxy, sek.-Butoxy oder tert.-Butoxy, und worin eine oder zwei CH₃-Gruppen durch NH₂, NAH, NA₂ oder CN ersetzt sein können, wie bspw. N, N'-Dimethylaminoalkyl, 2-Aminoethyl, 3-Aminopropyl, 4-Aminobutyl, 5-Aminopentyl, 3-Aminomethyl-cyclobutyl, Cyanoalkyl, (Isoindol-1,3-dion)2-yl oder (Carbamidsäure-tert.-butylester)-butyl.

Cycloalkyl bedeutet vorzugsweise Cyclopropyl, Cyclobutyl, Cylopentyl, Cyclohexyl oder Cycloheptyl.

Ar, Ar-A (Aryl-alkyl) bzw. A-Ar (Alkyl-aryl) bedeuten z.B. unsubstituiertes Phenyl, Naphthyl oder Biphenyl, weiterhin vorzugsweise z.B. durch A, Fluor, Chlor, Brom, Iod, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Benzyloxy, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl mono-, di- oder trisubstituiertes Phenyl, Naphthyl oder Biphenyl.

Ar, Ar-A bzw. A-Ar bedeuten weiterhin Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl, (4-Methoxy-phenyl)methyl, (3-Methoxy-phenyl)methyl, (4-Methoxy-phenyl)ethyl, (3-Methoxy-phenyl)ethyl.

Ar, Ar-A bzw. A-Ar bedeuten weiterhin vorzugsweise unsubstituiertes oder ein-, zwei- oder dreifach z.B. durch Carbonylsauerstoff, F, Cl, Br, Methyl, Ethyl, Propyl, Phenyl, Benzyl, -CH₂-Cyclohexyl, Hydroxy, Methoxy, Ethoxy, Amino, Methylamino, Dimethylamino, Nitro, Cyan, Carboxy, Methoxycarbonyl, Aminocarbonyl, Methylaminocarbonyl, Dimethylaminocarbonyl, Acetamino, Ureido, Methylsulfonylamino, Formyl, Acetyl, Aminosulfonyl und/oder Methylsulfonyl substituiertes 2-, 3- oder 4-Phenyl, 2-, 3- oder 4-Phenyl-methyl, 2-, 3- oder 4-Phenyl-ethyl, 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 3- oder 4-Pyridyl-methyl, 2-, 3- oder 4-Pyridyl-ethyl, 2-, 4-, 5- oder 6-Pyrimidinyl, 2-, 3-, 5-, oder 6-Pyrazin-1- oder 4-yl, weiterhin bevorzugt 1,2,3-Triazol-1-, -4-oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, 1-, 2-, 3-, 4-, 5-, 6- oder 7-Indolyl, 2-, 3-, 4- oder 5-Isoindolyl, 2-, 6, -oder 8-Purinyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 1-, 3-, 4-, 5-, 6-, 7- oder 8-Isochinolinyl, 3-, 4-, 5-, 6-, 7- oder 8-Chinolinyl, 2-, 4-, 5-, 6-, 7- oder 8-Chinazolinyl, 5- oder 6-Chinoxalinyl, 4-, 5-, oder 6-Phthalazinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Die heterocyclischen Reste können auch teilweise oder vollständig hydriert sein und bedeuten z.B. auch 2,3-Dihydro-2-, -3-, -4- oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder-4-pyrazolyl, 1,4-Dihydro-1-, -2-, -3- oder -4-pyridyl, 1,2,3,4-Tetrahydro-1-, -2-, -3-, -4-, -5- oder -6-pyridyl, 2-, 3-5- oder 6-Piperidin-1 oder 4-yl, 2-, 3- oder 4-Morpholinyl, Tetrahydro-2-, - 3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)-phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Die Bezeichnung "substituiert" bezieht sich vorzugsweise auf die Substitution mit den obengenannten Substituenten, wobei mehrere unterschiedliche Substitutionsgrade möglich sind, falls nicht anders angegeben.

Erfindungsgemäß sind auch alle physiologisch unbedenklichen Salze, Solvate und Stereoisomere dieser Verbindungen, einschließlich deren Mischungen in allen Verhältnissen.

Die Verbindungen der Formel I können ein oder mehrere chirale Zentren aufweisen. Sie können dementsprechend in verschiedenen enantiomeren Formen auftreten und in racemischer oder in optisch aktiver Form vorliegen. Gegenstand der Erfindung sind deshalb auch die optisch aktiven Formen (Stereoisomeren), die Enantiomeren, die Racemate, die Diastereomeren sowie Hydrate und Solvate dieser Verbindungen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktive Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wässrige oder alkoholische Lösungsmittelgemische wie z.B. Hexan/Isopropanol/ Acetonitril z.B. im Verhältnis 82:15:3.
Eine elegante Methode zur Spaltung von Racematen mit Estergruppen (z.B. Acetylester) stellt die Verwendung von Enzymen, insbesondere Esterasen, dar.

Vorzugsweise ist bei den Verbindungen der Formel I die Gruppierung 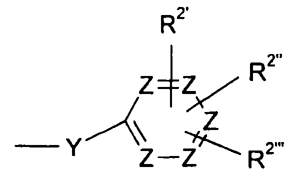 über die 2- oder 3-Position entsprechend der Indol-Nomenklatur mit der von Indol bzw. 2,3-Dihydroindol abgeleiteten Gruppierung verknüpft.

Eine bevorzugte Gruppe von Verbindungen der Formel I entspricht der Formel Ia 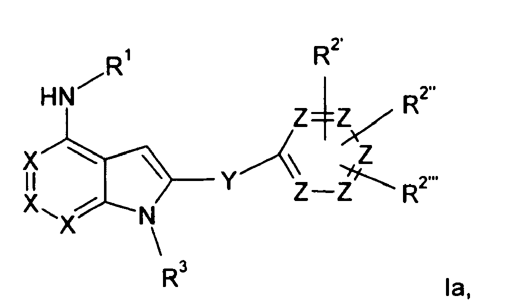 worin R¹, R², R³, Y und Z die für die Formel I angegebene Bedeutung haben sowie ihre pharmazeutisch unbedenklichen Salze, Solvate und Stereoisomere, einschließlich deren Mischungen in allen Verhältnissen.

Eine weiter bevorzugte Gruppe von Verbindungen der Formel Ia entspricht der Formel AII 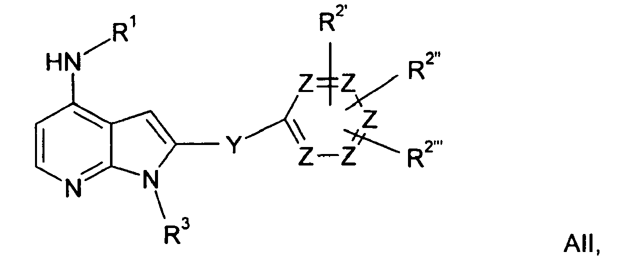 worin R¹, R², R³, Y und Z die für die Formel I bzw. Ia angegebene Bedeutung haben sowie ihre pharmazeutisch unbedenklichen Salze, Solvate und Stereoisomere, einschließlich deren Mischungen in allen Verhältnissen.

Eine weitere bevorzugte Gruppe von Verbindungen der Formel AII entspricht der Formel AII 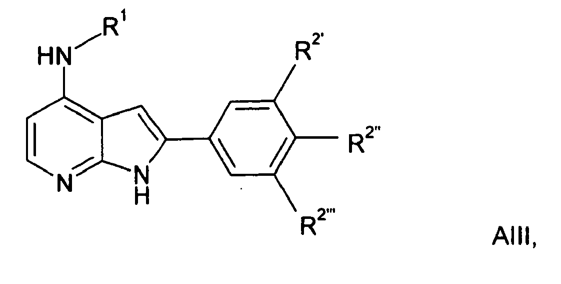 worin Y eine Bindung, Z CH, R³ H bedeutet und R¹ bzw. R² die für die Formel I, Ia bzw. AII angegebene Bedeutung haben sowie ihre pharmazeutisch unbedenklichen Salze, Solvate und Stereoisomere, einschließlich deren Mischungen in allen Verhältnissen.

Weiter bevorzugte Untergruppen von Verbindungen der Formel I, Ia, AII und AIII können durch die folgenden Teilformeln Aa bis Ag ausgedrückt werden, die den Formeln I, Ia, AII bzw. AIII entsprechen, worin jedoch
bei der Teilformel Aa
- R¹: unsubstituiertes oder ein- oder mehrfach durch Hal, Cyano, Me thyl, CHal₃ oder Methoxy substituiertes Phenyl, Phenyl-carbonyl, Phenylmethyl, Pyridyl, Pyridylmethyl, Pyridylethyl, Pyrimidyl, Pipe razyl, Chinoliny, Imidazoyl, Imidazyolpropyl, Pyrrolyl, Pyrrolylethyl, weiterhin H, N,N'-Dimethylaminopropyl oder Cyanobutyl oder einen der folgenden Reste 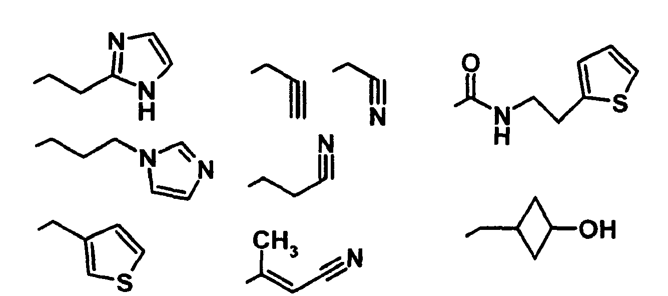 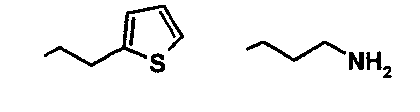 wobei die Verknüpfung mit dem Grundkörper der Formeln I, Ia, AII bzw. AIII jeweils über die nach links stehende Bindung, die keine Methylguppe ist, erfolgt,
bedeutet
und R², R³ die für die Formel I angegebene Bedeutung haben,
bei der Teilformel Ab
- R^{2'}, R^{2"}, R^{2'"}: jeweils unabhängig voneinander H, Methoxy, Ethoxy, n- Propoxy, i-Propoxy, 2-Phenyl-ethoxy, 3-Phenyl-propoxy oder 4-Phenyl-butoxy bedeuten
und R¹, R³ die für die Formel I angegebene Bedeutung haben,
bei der Teilformel Ac
- R^{2'}, R^{2"}, R^{2"'}: jeweils unabhängig voneinander H, Methoxy oder 3-Phenyl- propoxy bedeuten
und R¹, R³ die für die Formel I angegebene Bedeutung haben,
bei der Teilformel Ad
- R^{2'}, R^{2"}, R^{2"'}: jeweils Methoxy bedeuten
und R¹, R³ die für die Formel I angegebene Bedeutung haben,
bei der Teilformel Ae
- R^{2'}, R^{2"}, R^{2"'}: jeweils unabhängig voneinander H, Methoxy, Ethoxy, n- Propoxy, i-Propoxy, 2-Phenyl-ethoxy, 3-Phenyl-propoxy oder 4-Phenyl-butoxy bedeuten
und R¹, R³ die für die Teilformel Aa angegebene Bedeutung haben,
bei der Teilformel Af
- R^{2'}, R^{2"}, R^{2"'}: jeweils unabhängig voneinander H, Methoxy oder 3-Phenyl- propoxy bedeuten
und R¹, R³ die für die Teilformel Aa angegebene Bedeutung haben,
bei der Teilformel Ag
- R^{2'}, R^{2"}, R^{2'"}: jeweils Methoxy bedeuten
und R¹, R³ die für die Teilformel Aa angegebene Bedeutung haben
sowie ihre pharmazeutisch unbedenklichen Salze, Solvate und Stereoisomere, einschließlich deren Mischungen in allen Verhältnissen.

Eine weitere bevorzugte Gruppe von Verbindungen der Formel I entspricht der Formel BII 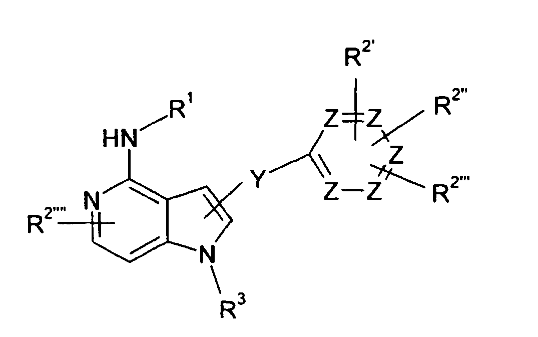 worin R¹, R², R³, Y und Z die für die Formel I angegebene Bedeutung haben sowie ihre pharmazeutisch unbedenklichen Salze, Solvate und Stereoisomere, einschließlich deren Mischungen in allen Verhältnissen.

Eine weiter bevorzugte Gruppe von Verbindungen der Formel BII entspricht der Formel BIII 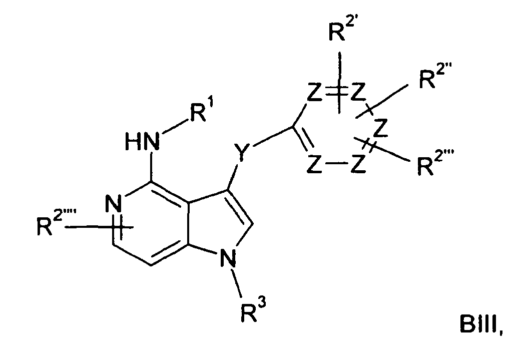 worin sämtliche Reste die für die Formel BII angegebene Bedeutung haben.

Eine noch weiter bevorzugte Gruppe von Verbindungen der Formel BIII entspricht der Formel BIV 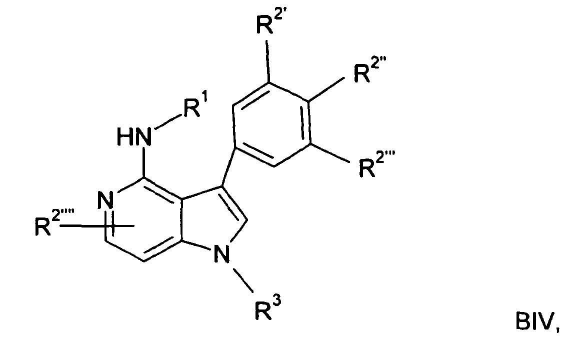 worin Y eine Bindung, Z CH bedeutet und R¹, R² bzw. R³ die für die Formel I, BII bzw. BIII angegebene Bedeutung haben sowie ihre pharmazeutisch unbedenklichen Salze, Solvate und Stereoisomere, einschließlich deren Mischungen in allen Verhältnissen.

Eine noch stärker bevorzugte Gruppe von Verbindungen der Formel BIV entspricht der Formel BV 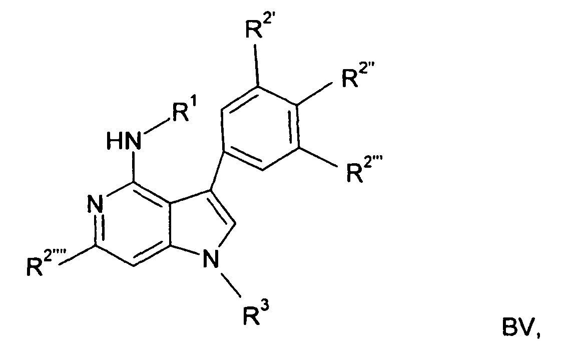 worin sämtliche Reste die für die Formel BIV angegebene Bedeutung haben sowie ihre pharmazeutisch unbedenklichen Salze, Solvate und Stereoisomere, einschließlich deren Mischungen in allen Verhältnissen.

Weiter bevorzugte Untergruppen von Verbindungen der Formel I, BII, BIII BIV und BV können durch die folgenden Teilformeln Ba bis Bg ausgedrückt werden, die den Formeln I, BII, BIII, BIV bzw. BV entsprechen, worin jedoch
bei der Teilformel Ba
- R¹: unsubstituiertes oder ein- oder mehrfach durch Hal, Cyano, Methyl oder Methoxy substituiertes Phenyl, Phenylmethyl, Pyridyl, Pyridyl methyl, Pyridylethyl, Pyrimidyl, Piperazyl, Chinoliny, Imidazoyl, Imi dazyolpropyl, Pyrrolyl, Pyrrolylethyl sowie H bedeutet
und R² bzw. R³ die für die Formel I angegebene Bedeutung haben,
bei der Teilformel Bb
- R^{2'}, R^{2"}, R^{2'"}: jeweils unabhängig voneinander H, Methoxy, Ethoxy, n- Propoxy, i-Propoxy, Phenyl-methoxy oder Phenyl-ethoxy bedeuten,
- R^{2""}: H, Hal oder NH₂
und R¹ bzw. R³ die für die Formel I angegebene Bedeutung haben,
bei der Teilformel Bc
einer der Reste R^{2'}, R^{2"}, R^{2'"} Methoxy oder Phenyl-methoxy und die anderen beiden H bedeuten
und R¹ bzw. R³ die für die Formel I angegebene Bedeutung haben,
bei der Teilformel Bd
- R³: 2-Aminoethyl, 3-Aminopropyl, 4-Aminobutyl, 5-Aminopentyl, 3- Aminomethyl-cyclobutyl, (Isoindol-1,3-dion)2-yl oder 4-(Carbamid säure-tert.-butylester)but-1-yl bedeutet
und R¹ bzw. R² die für die Formel I angegebene Bedeutung haben,
bei der Teilformel Be
- R^{2'}, R^{2"}, R^{2""}: jeweils unabhängig voneinander H, Methoxy, Ethoxy, n- Propoxy, i-Propoxy, Phenyl-methoxy oder Phenyl-ethoxy,
- R^{2""}: H, Cl oder NH₂,
- R¹: unsubstituiertes oder ein- oder mehrfach durch Hal, Cyano, Methyl oder Methoxy substituiertes Phenyl, Phenylmethyl, Pyridyl, Pyridylmethyl, Pyridylethyl, Pyrimidyl, Piperazyl, Chi noliny, Imidazoyl, Imidazyolpropyl, Pyrrolyl, Pyrrolylethyl so wie H und
- R³: 2-Aminoethyl, 3-Aminopropyl, 4-Aminobutyl, 5-Aminopentyl, 3-Aminomethyl-cyclobutyl, (Isoindol-1,3-dion)2-yl oder 4-(Car bamidsäure-tert.- butylester)but-1-yl bedeutet,
bei der Teilformel Bf
einer der Reste R^{2'}, R^{2"}, R^{2'"} Methoxy oder Phenyl-methoxy und die anderen beiden H bedeuten
- R¹: H, Pyridyl, Pyridylmethyl oder (4-Methoxy-phenyl)methyl und
- R³: 2-Aminoethyl, 3-Aminopropyl, 4-Aminobutyl, 5-Aminopentyl, 3-Aminomethyl-cyclobutyl, (Isoindol-1,3-dion)2-yl oder 4-(Car bamidsäure-tert.- butylester)but-1-yl bedeutet,
bei der Teilformel Bg
einer der Reste R^{2'}, R^{2"}, R^{2'"} Methoxy oder Phenyl-methoxy und die anderen beiden H bedeuten
- R¹: H, Pyrid-2 oder 3-yl, Pyrid-2 oder 3-yl-methyl oder (4- Methoxy-phenyl)methyl und
- R³: 2-Aminoethyl, 3-Amino-n-propyl, 4-Amino-n-butyl, 5-Amino-n- pentyl, 3-Aminomethyl-cyclobut-1-yl, (Isoindol-1,3-dion)2-yl oder 4-(Carbamidsäure-tert.- butylester)but-1-yl bedeutet
sowie ihre pharmazeutisch unbedenklichen Salze, Solvate und Stereoisomere, einschließlich deren Mischungen in allen Verhältnissen.

Besonders bevorzugt sind Verbindungen, ausgewählt aus den in der Tabelle 1 aufgeführten Verbindungen sowie ihre pharmazeutisch unbedenklichen Salze, Solvate und Stereoisomere, einschließlich deren Mischungen in allen Verhältnissen.

Die in Tabelle 1 aufgeführten Verbindungen 27-31, 33, 37, 38, 39, 40, 41, 43, 46, 49-51, 56, 57, 67, 70, 72 und 74-88 sind nicht erfindungsgemäß, sondern dienen lediglich der Illustration.

Die in der Tabelle 1 angegebenen Schmelzpunkte beziehen sich, sofern kein Anion angegeben ist, auf die freie Base. In denjenigen Fällen, wo der Schmelzpunkt aufgrund von Zersetzung nicht bestimmt werden kann, ist eine Zersetzungstemperatur angegeben. Kann eine Verbindung nicht kristallin erhalten werden, ist die Materialbeschaffenheit bei Raumtemperatur angegeben (Öl bzw. Harz).

**Tabelle 1**

| | | IC50 (µM), IGF1R | Schmelzpunkt |
|---|---|---|---|
| 1 | 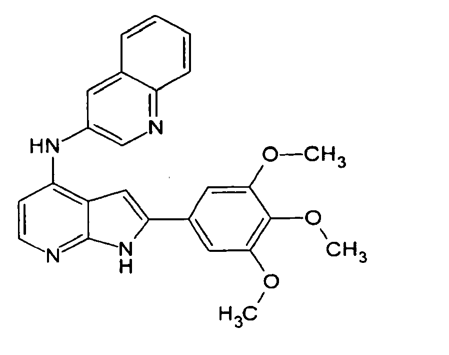 | 14 | 227-228,5°C (Dihydrochlorid) |
| 2 | 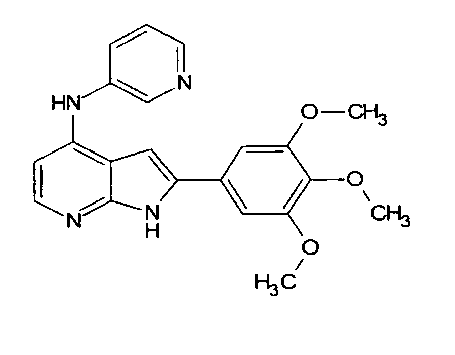 | 0,76 | 202-203°C (Dihydrochloridhydrat) |
| 3 | 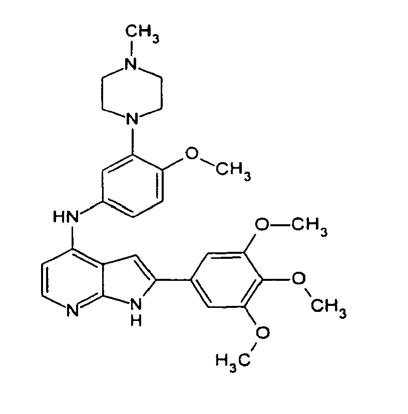 | 0,99 | 78-80°C (Trihydrochloridtrihydrat) |
| 4 | 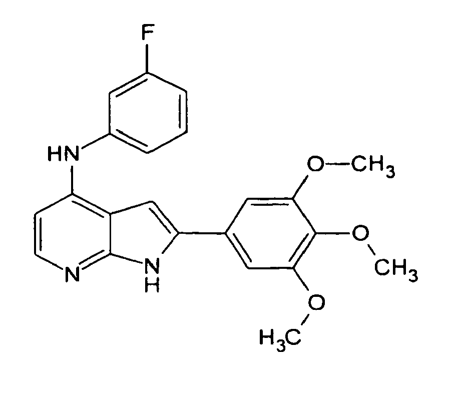 | 0,86 | 80-81°C |
| 5 | 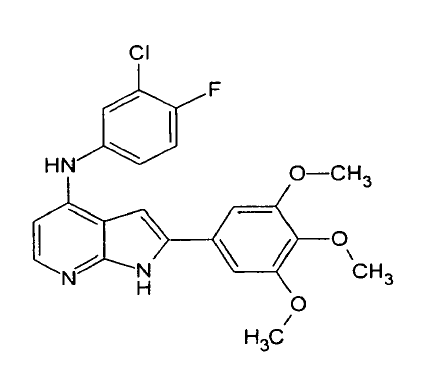 | | 267,5-269°C (Hydrochlorid) |
| 6 | 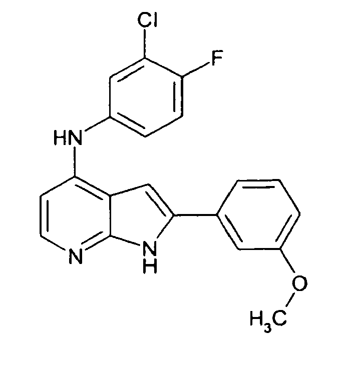 | | 164-165°C (Hydrochlorid) |
| 7 | 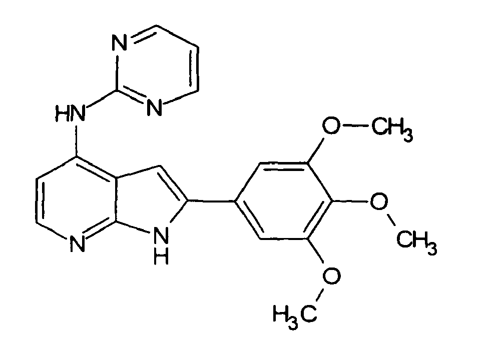 | 0,76 | 292-294°C (Dihydrochloridhydrat) |
| 8 | 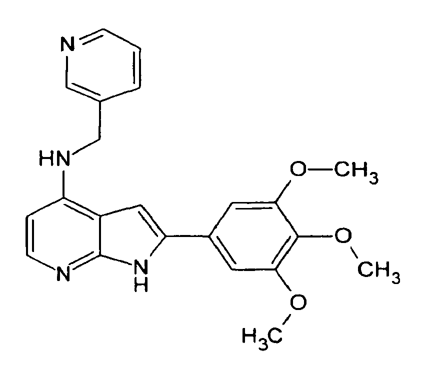 | 0,93 | 181,5-183°C (Dihydrochlorid-Dihydrat) |
| 9 | 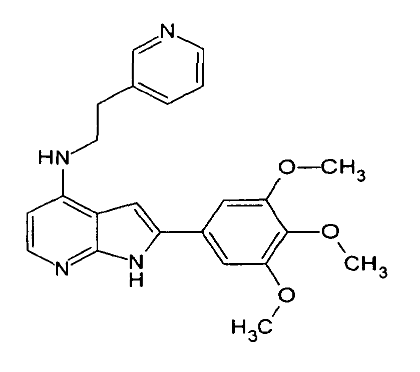 | 1,2 | 105,5-106,5°C (Dihydrochlorid-Dihydrat) |
| 10 | 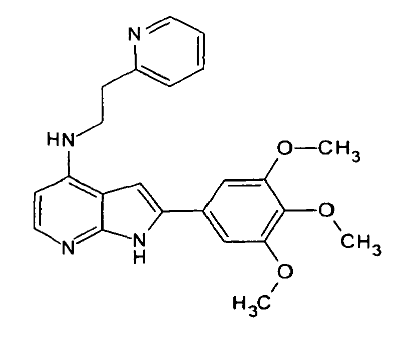 | 1,4 | 153-154°C (Dihydrochlorid-Dihydrat) |
| 11 | 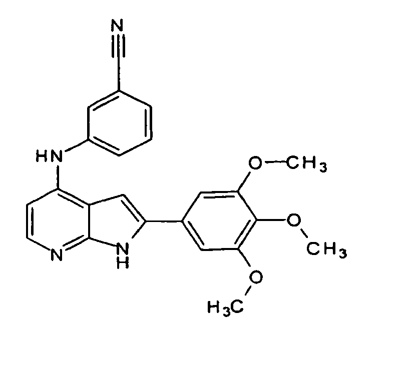 | 1,6 | 291,0-292,5°C (Hydrochlorid-Dihydrat) |
| 12 | 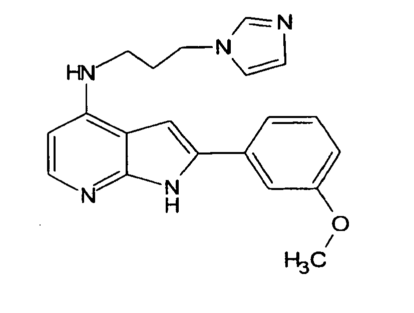 | | 161-162°C (Dihydrochlorid) |
| 14 | 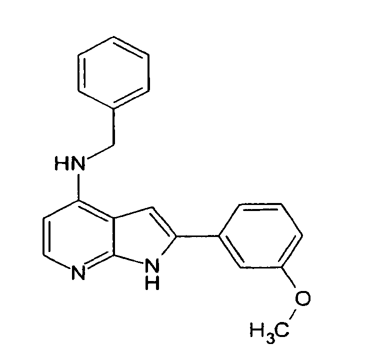 | 10 | 117-119°C (Hydrochlorid) |
| 15 | 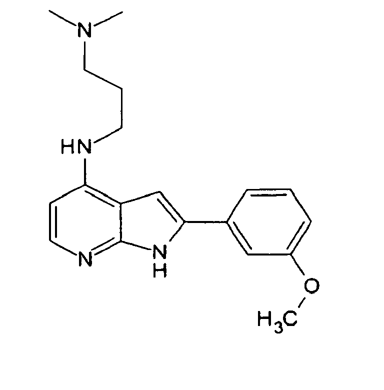 | | 257-258°C (Dihydrochlorid-Hydrat) |
| 16 | 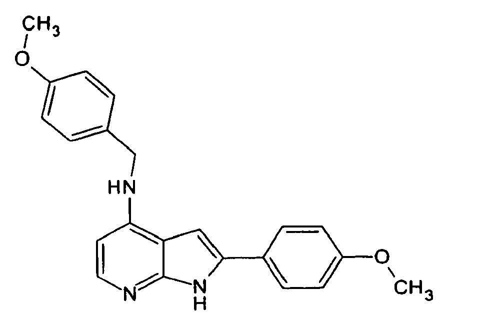 | 11 | 196-197°C (Hydrochloride) |
| 17 | 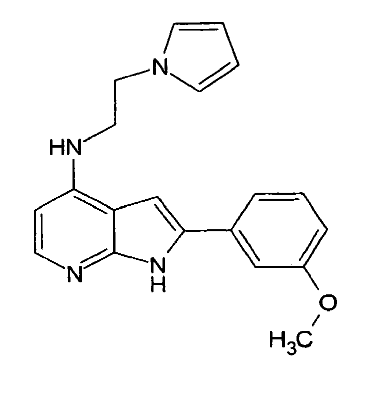 | | 218 - 219°C (Hydrochlorid) |
| 18 | 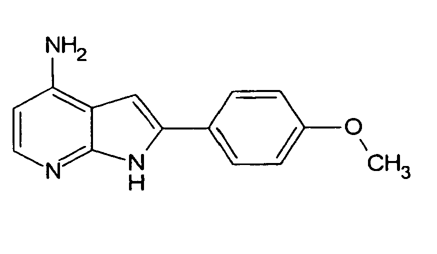 | | |
| 19 | 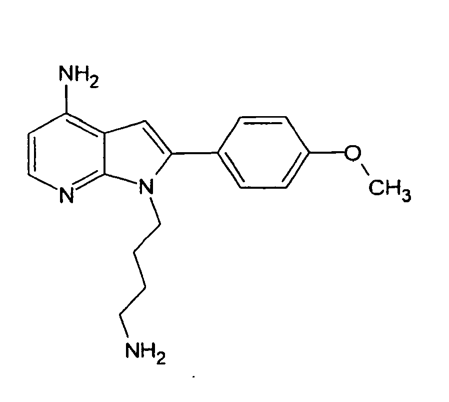 | | |
| 20 | 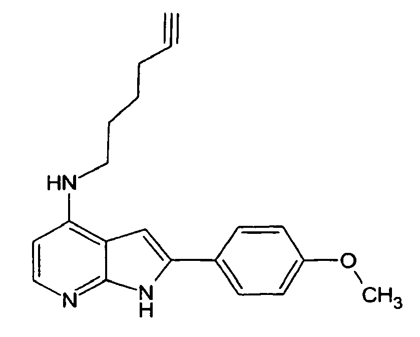 | 9,9 | 118-120°C (Hydrochlorid Hydrat) |
| 21 | 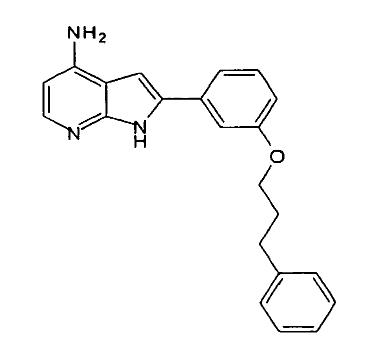 | | Öl |
| 22 | 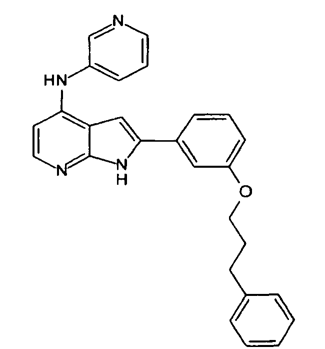 | | 166-167,5°C (2HCl) |
| 23 | 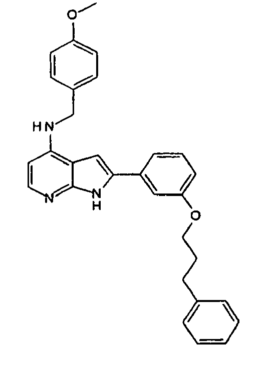 | | 164-165°C |
| 24 | 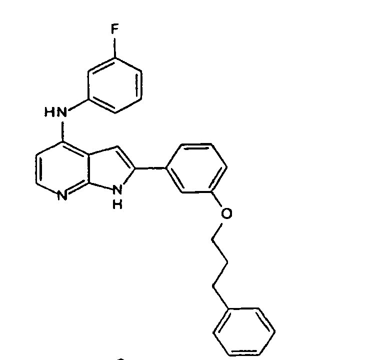 | | 210-211,5°C (HCl) |
| 25 | 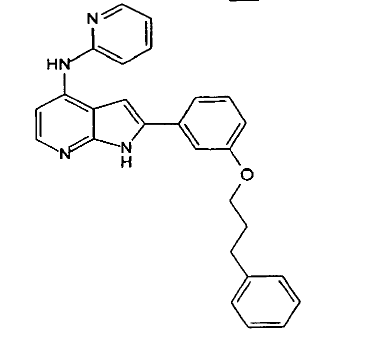 | | 175-176°C (HCl) |
| 26 | 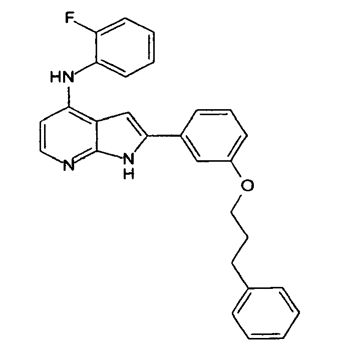 | | 155-156°C (HCl) |
| 27 | 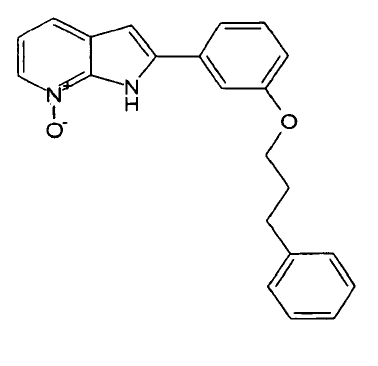 | | |
| 28 | 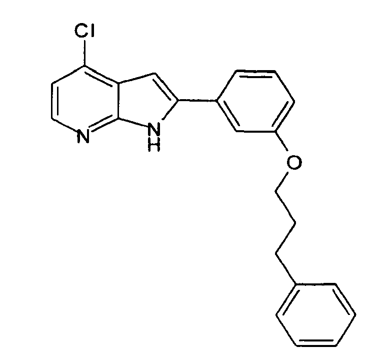 | | |
| 29 | 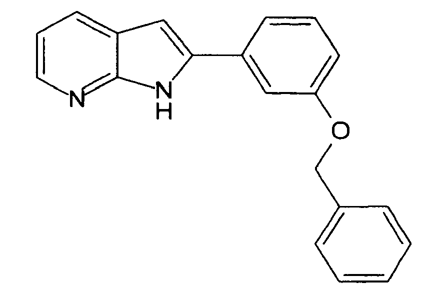 | | |
| 30 | 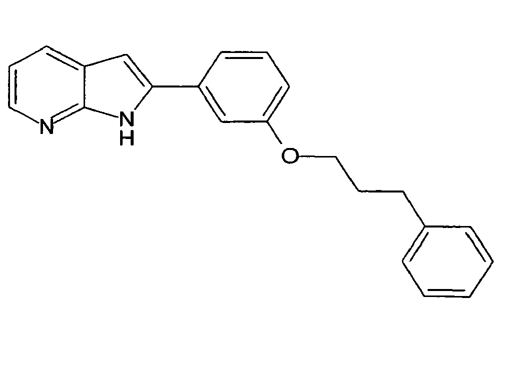 | | 128-129°C |
| 31 | 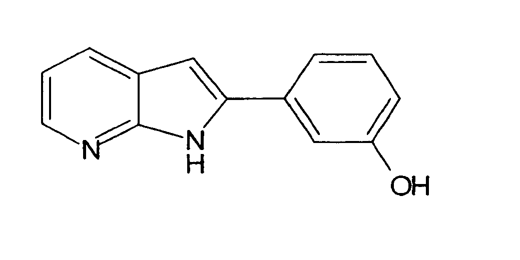 | | 210,5-211°C |
| 32 | 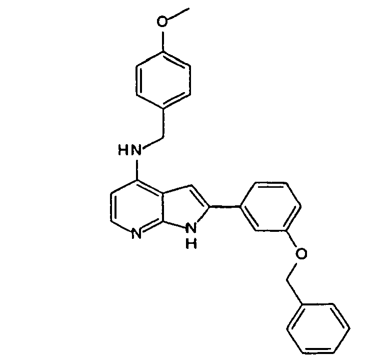 | | 218-219°C |
| 33 | 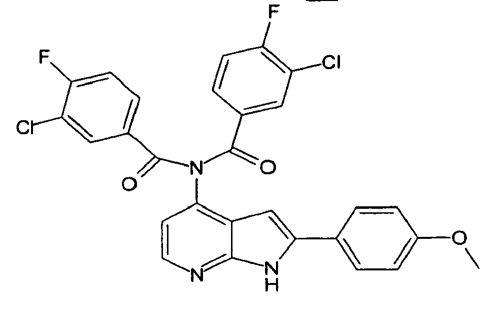 | | 142-144°C (TFA) |
| 34 | 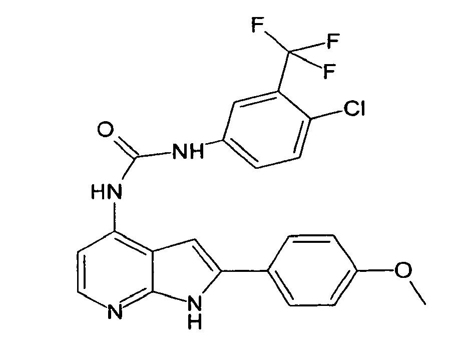 | | 141-142°C (TFA) |
| 35 | 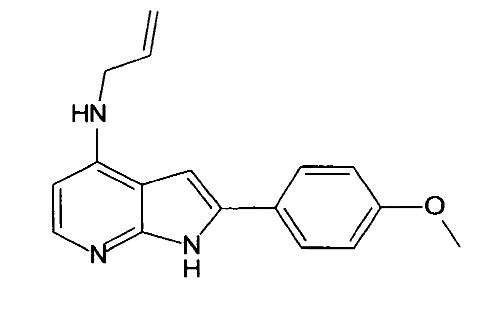 | | 189,5-190°C (TFA) |
| 36 | 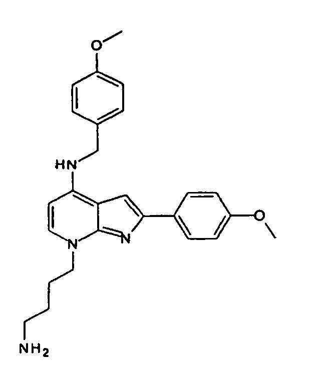 | | 127-127,5°C (TFA) |
| 37 | 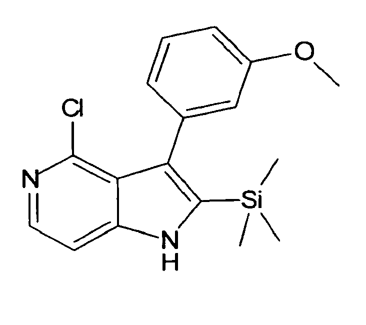 | | 137°C |
| 38 | 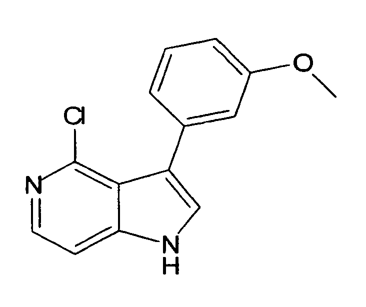 | | Öl |
| 39 | 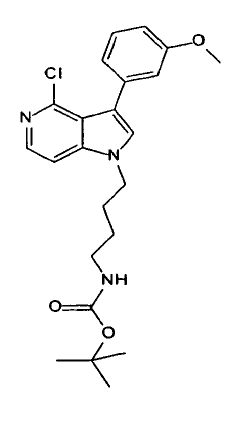 | | Öl |
| 40 | 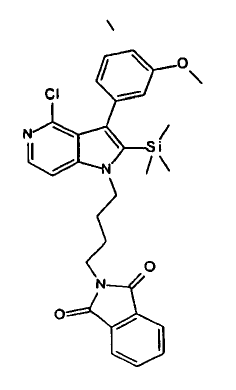 | | 183-184°C (TFA) |
| 41 | 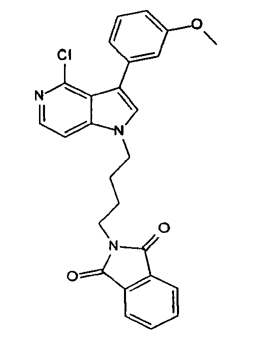 | | Öl |
| 42 | 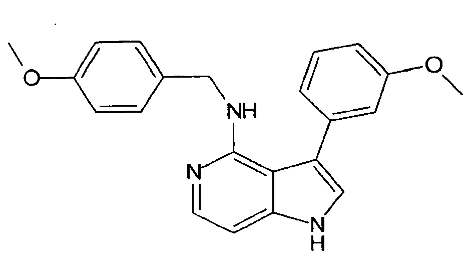 | | 226-227°C (HCl) |
| 43 | 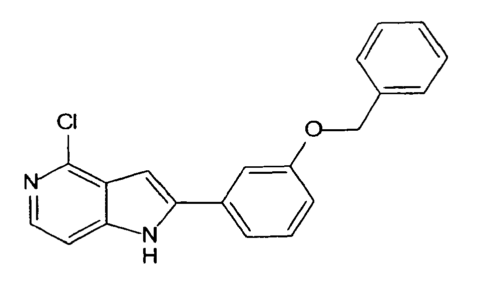 | | Öl |
| 44 | 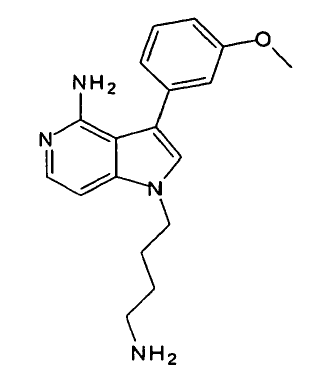 | | Harz |
| 45 | 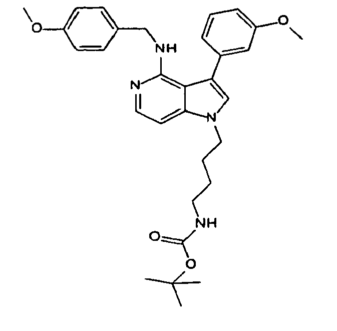 | | Öl |
| 46 | 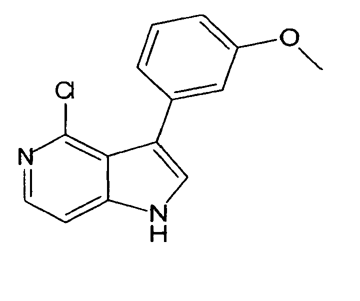 | | 159,5-160°C |
| 47 | 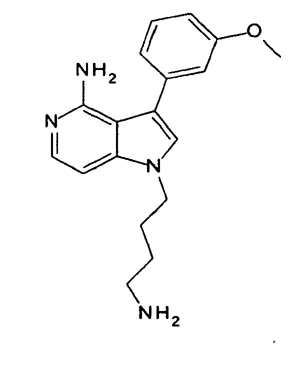 | | 50°C (TFA) |
| 48 | 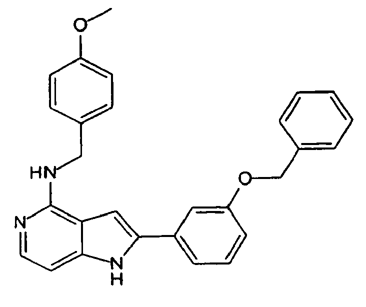 | | 130-131°C (TFA) |
| 49 | 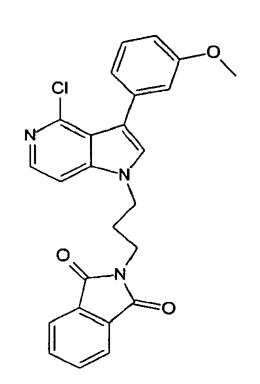 | | 129-130°C (TFA) |
| 50 | 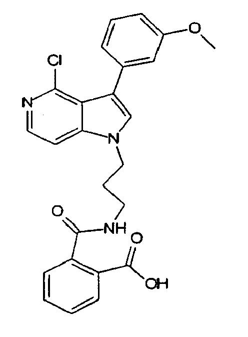 | | 122-124°C |
| 51 | 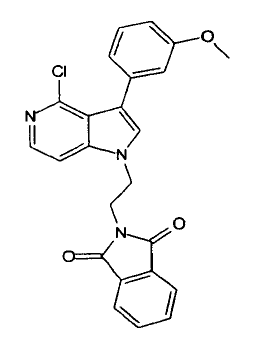 | | 130°C (TFA, Zersetzung) |
| 52 | 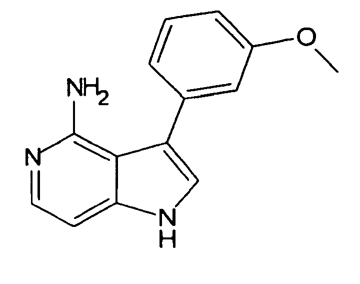 | | Öl |
| 53 | 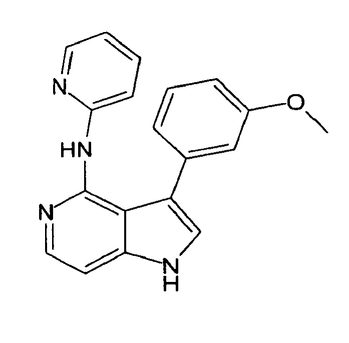 | | >299°C |
| 54 | 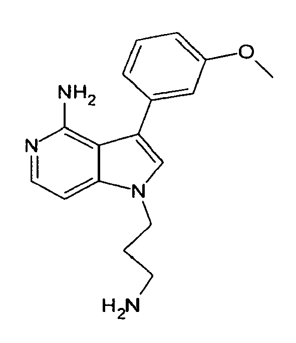 | | Öl |
| 55 | 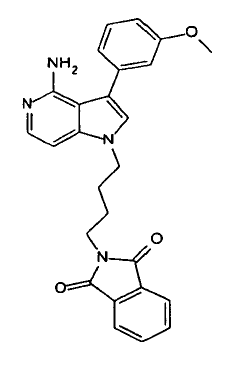 | | 188-189°C (TFA) |
| 56 | 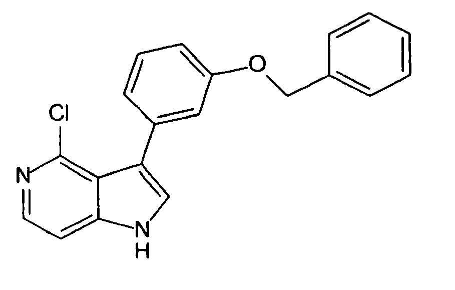 | | 156-158°C |
| 57 | 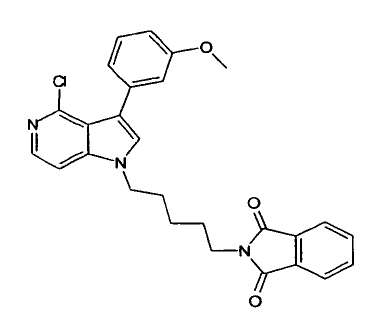 | | Harz |
| 58 | 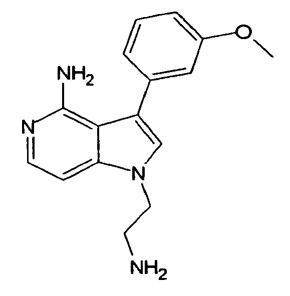 | | Öl |
| 59 | 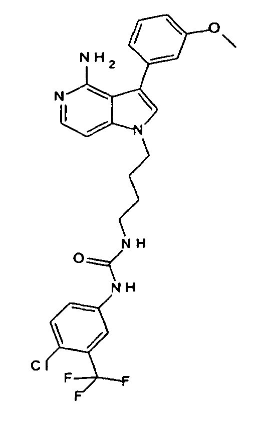 | | Öl |
| 60 | 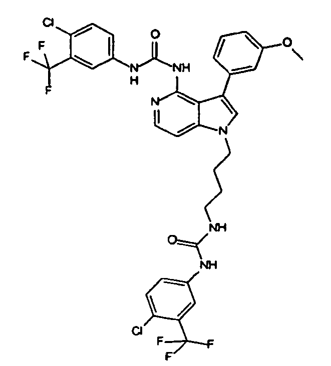 | | Öl |
| 61 | 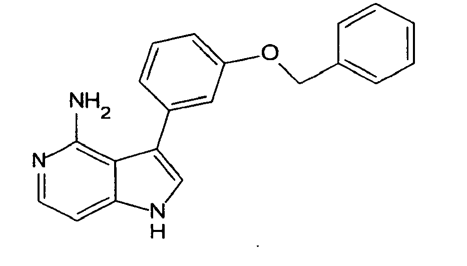 | | Harz |
| 62 | 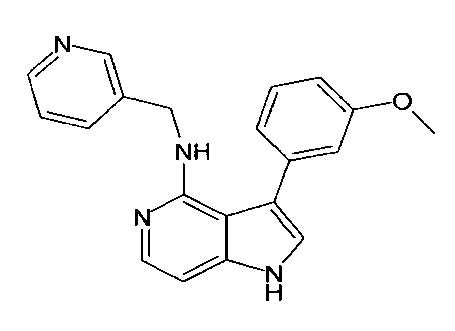 | | 80°C (Zersetzung) |
| 63 | 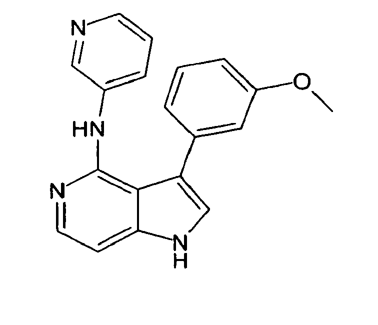 | | 177-178°C |
| 64 | 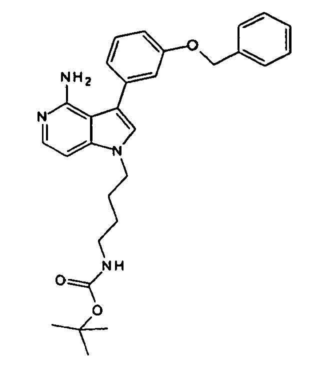 | | Harz (TFA) |
| 65 | 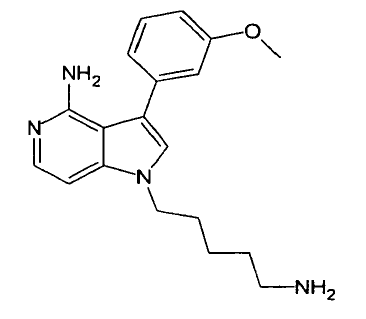 | | Harz (TFA) |
| 66 | 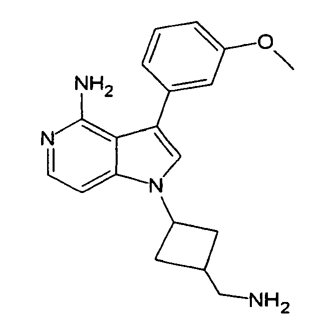 | | Harz (TFA) |
| 67 | 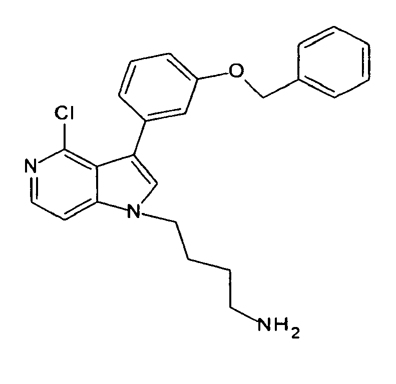 | | Öl (TFA) |
| 68 | 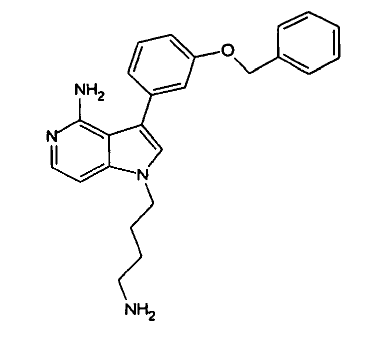 | 20 | Öl |
| 69 | 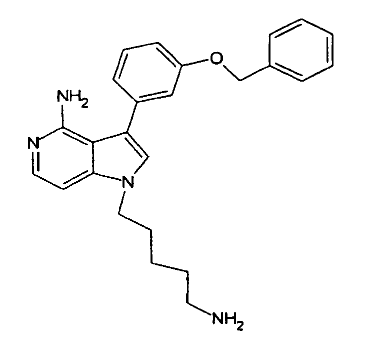 | 5,7 | 75-78°C |
| 70 | 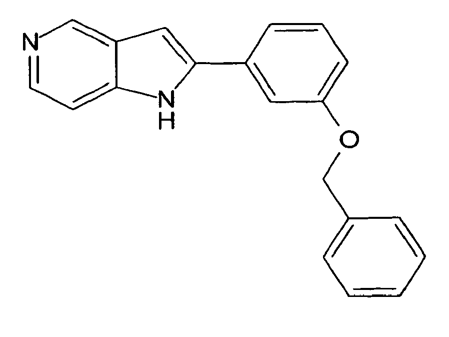 | | 168-170°C |
| 71 | 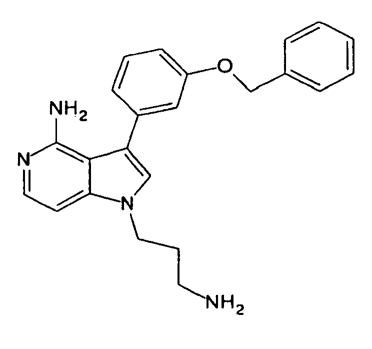 | 4,8 | Öl |
| 72 | 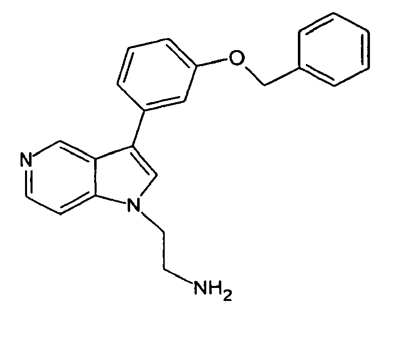 | 25 | |
| 73 | 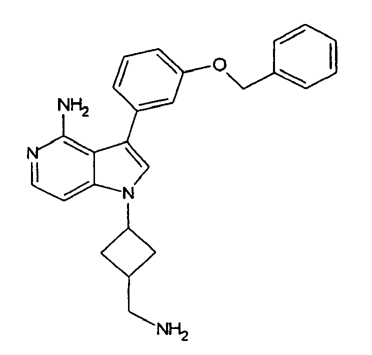 | 0,91 | |
| 74 | 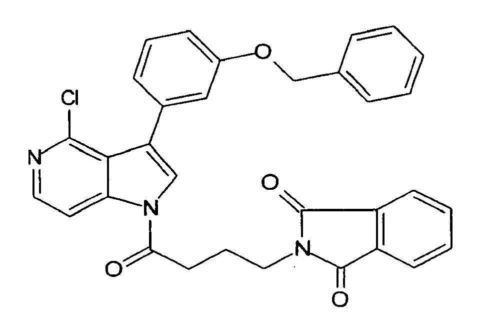 | | |
| 75 | 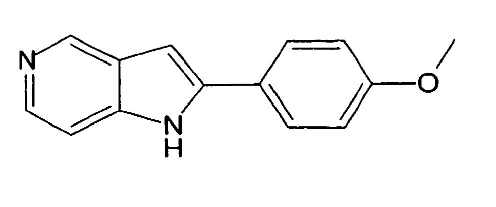 | | 205-207°C |
| 76 | 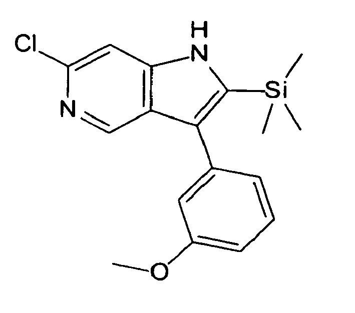 | | 179-179,5°C |
| 77 | 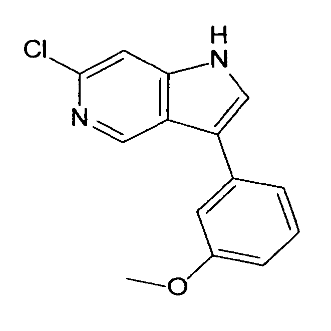 | | 211-212°C |
| 78 | 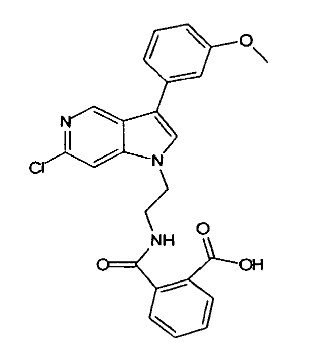 | | 160°C (Zersetzung) |
| 79 | 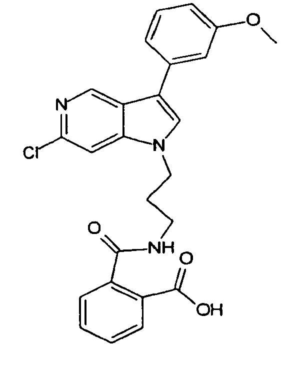 | | 100-101°C |
| 80 | 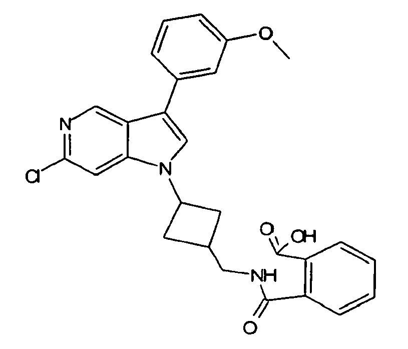 | | 177-180°C |
| 81 | 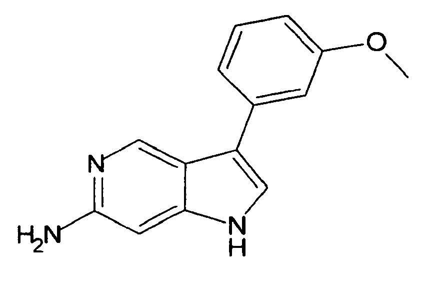 | | Öl |
| 82 | 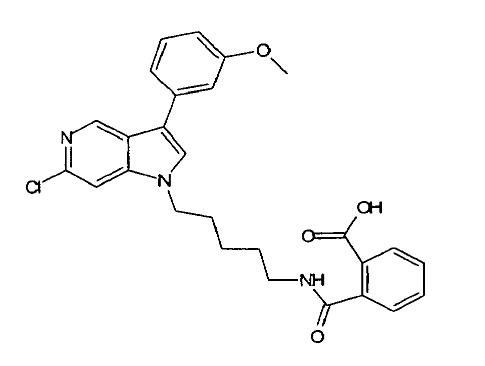 | | 97-99°C |
| 83 | 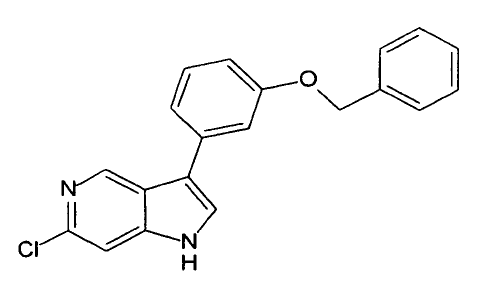 | | 225-228,5°C |
| 84 | 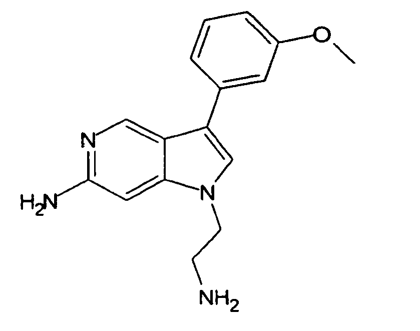 | | Harz |
| 85 | 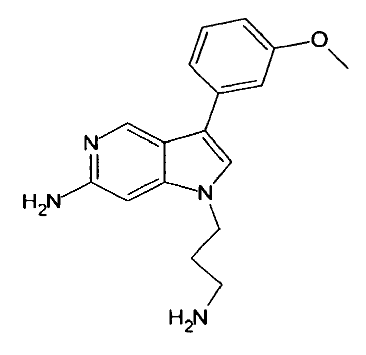 | | Harz |
| 86 | 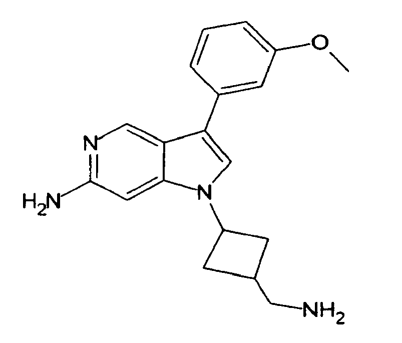 | | Öl |
| 87 | 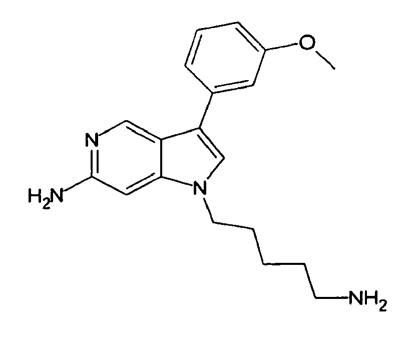 | | Öl |
| 88 | 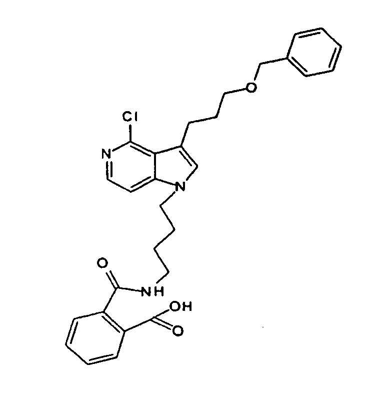 | | Öl |

Als Säureadditionssalze kommen anorganische oder organische Salze aller physiologisch oder pharmakologisch unbedenklichen Säuren in Frage, beispielsweise Halogenide, insbesondere Hydrochloride oder Hydrobromide, Lactate, Sulfate, Citrate, Tartrate, Maleate, Fumarate, Oxalate, Acetate, Phosphate, Methylsulfonate oder p-Toluolsulfonate.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind beispielsweise Hydrate, wie Monohydrate oder Dihydrate oder Alkoholate, d.h. Additionsverbindungen mit Alkoholen wie beispielsweise mit Methanol oder Ethanol.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder angestrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder Verhinderung von Nebenwirkungen oder auch die Verminderung des Fortschreitens einer Krankheit, eines Leidens oder einer Störung. Die Bezeichnung "therapeutisch wirksame Menge" umfasst auch die Mengen, die wirkungsvoll sind, die normale physiologische Funktion zu erhöhen.

Gegenstand der Erfindung sind auch Mischungen der erfindungsgemäßen Verbindungen der Formel I, z.B. Gemische zweier Diastereomere z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Weiterhin betrifft die vorliegende Erfindung ein Verfahren zur Herstellung von Verbindungen der Formel I sowie ihrer physiologisch unbedenklichen Salze, Solvate und Stereoisomere, dadurch gekennzeichnet, dass man in einem ersten Schritt eine Verbindung der Formel XI 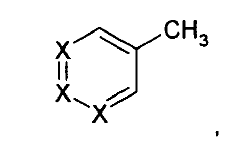 worin X die oben angegebenen Bedeutung hat mit einer Verbindung der Formel VIII 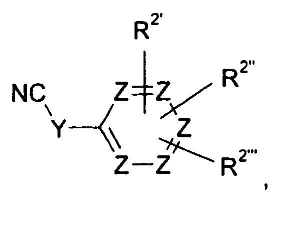 worin Z, Y und R² die oben angegebenen Bedeutungen haben, zu einer Verbindung der Formel VII umsetzt 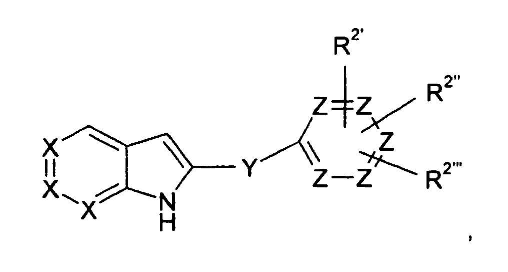 die ggf. zu einer entsprechenden 2,3-Dihydro-Indolverbindung reduziert wird und aus der dann im nächsten Schritt eine Verbindung der Formel VI 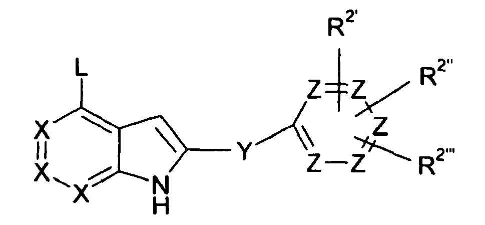 hergestellt wird, worin L eine Abgangsgruppe wie bspw. Cl, Br, I, Mesylat, Tosylat, Phenylsulfonat oder Trifluoracetat ist,
und die Verbindung der Formel VI dann in einem weiteren Schritt mit einer Verbindung der Formel V

R¹-NH₂

zur Reaktion gebracht wird, um eine Verbindung der Formel IV 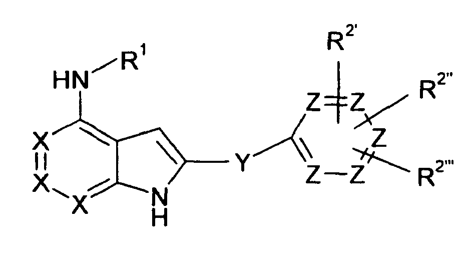 zu erhalten, die schließlich mit einem Rest R³ zu einer Verbindung der Formel I verknüpft wird,
und ggf. eine Base oder Säure der Formel I in eines ihrer Salze umwandelt.

Bei einem weiteren erfindungsgegenständlichen Verfahren zur Herstellung von Verbindungen der Formel I sowie ihrer physiologisch unbedenklichen Salze, Solvate und Stereoisomere setzt man in einem ersten Schritt eine Verbindung der Formel (ix) 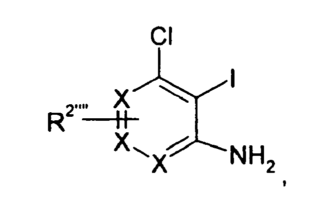 worin X die bei der Formel I angegebene Bedeutung hat, mit einem Arylsilyl-acetylen der Formel (viii) 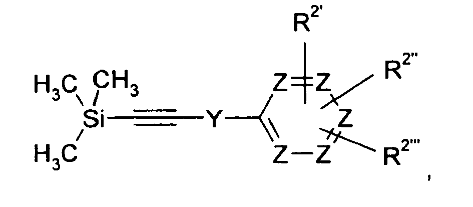 worin Z, Y und R² die bei der Formel I angegebenen Bedeutungen haben, zu einer Verbindung der Formel (vii) 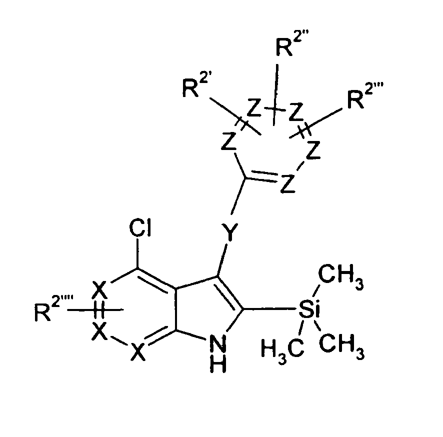 um, die ggf. zu einer entsprechenden 2,3-Dihydro-Indolverbindung reduziert wird, die dann in einem nächsten Schritt zu einer Verbindung der Formel (vi) 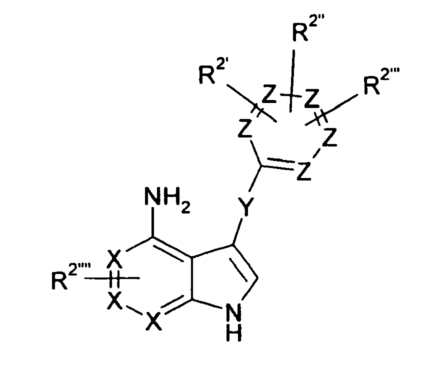 umgewandelt wird, an die, falls gewünscht, noch ein Rest R¹ und/oder ein Rest R³ unter Erhalt einer Verbindung der Formel (iii) 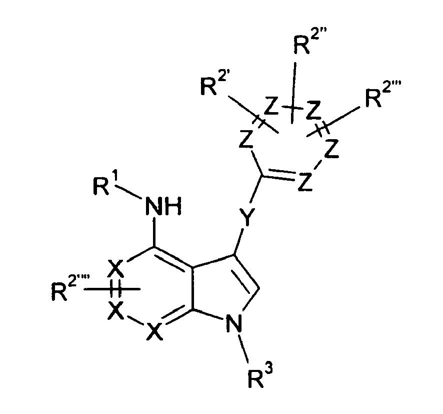 angefügt wird, und wandelt ggf. eine Base oder Säure der Formel (iii) in eines ihrer Salze um.

Sollen Verbindungen der Formel (ii) erhalten werden, bei denen der bizyklische Arylkörper über die 2-Position mit dem monozyklischen Arylkörper verknüpft sind 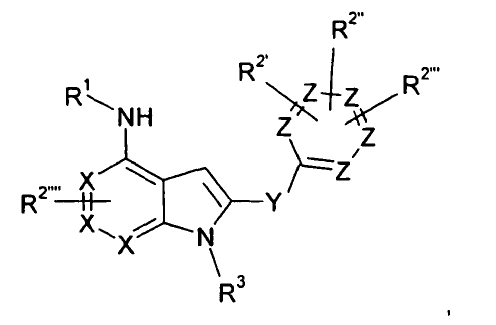 setzt man im ersten Schritt ein Arylactylen ohne Silylgruppe ein.

Die Ausgangsstoffe für die beiden Verfahrensvarianten sind in der Regel bekannt. Sind sie neu, so können sie nach an sich bekannten Methoden hergestellt werden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York) beschrieben sind.

Die Verbindungen der Formel I und auch die Ausgangsstoffe zu ihrer Herstellung werden nach an sich bekannten Methoden hergestellt, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Aza-Heterocyclen der Formel I können vorzugsweise erhalten werden, indem man wie folgt vorgeht:
a) Eine Verbindung der Formel IX wird analog Davis et al. (Tetrahedron 1992, 48 (5), 939 - 952) bei tiefer Temperatur in einem inerten Lösungsmittel vorgelegt. Anschließend wird eine Verbindung der Formel VIII zugeben und das Reaktionsgemisch gerührt. Nach Beendigung der Reaktion wird das Reaktionsgemisch aufgereinigt und das Produkt als Feststoff, vorzugsweise kristallin, isoliert. An diesen Schritt kann sich wahlweise in die Reduzierung des gebildeten Aza-Indolderivats nach an sich bekannten Methoden (z.B. durch selektive Hydrierung) zu einem 2,3-Dihydroindolderivat anschließen.
b) Das Reaktionsprodukt aus Schritt (a) wird analog Chou et al. (J. Phys. Chem. A 2003, 107, 1459 - 1471) an der 4-Position des Indolkörpers mit einer Abgangsgruppe (z.B. Cl) versehen.
c) Das Reaktionsprodukt aus Schritt (b) wird bei erhöhter Temperatur mit einem Amin der Formel V zur Reaktion gebracht. Das Produkt dieser Reaktion, der gewünschte Aza-Heterozyklus der Formel I, wird aufgereinigt und aus dem Reaktionsgemisch abgetrennt.

Gemäß der alternativen Verfahrensvariante (siehe oben) wird eine Verbindung der Formel (vii) durch Umsetzen einer Verbindung der Formel (ix) mit einer Verbindung der Formel (viii) nach Larock (J. Am. Chem. Soc. 113:6689, 1991) bei hoher Temperatur in einem inerten Lösungsmittel unter Schutzgas erhalten, wobei die Verknüpfung zwischen bizyklischem und monozyklischem Arylkörper durch die Anwesenheit oder Abwesenheit einer Silylgruppe in der Verbindung der Formel (viii) gesteuert werden kann.
Die weitere Umsetzung erfolgt entsprechend Schritt (c).

Die zuvor beschriebenen Umsetzungen erfolgen in der Regel in einem inerten Lösungsmittel. Als inerte Lösungsmittel für die zuvor beschriebenen Umsetzungen eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Ether wie Diethylether, Diisopropylether, Tetrahydrofuran (THF) oder Dioxan; Glykolether wie Ethylenglykolmonomethyl- oder monoethylether (Methylglykol oder Ethylglykol), Ethylenglykoldimethylether (Diglyme); Ketone wie Aceton oder Butanon; Amide wie Acetamid, N-Methylpyrrolidon (NMP), Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Bevorzugt sind Sulfoxide wie Dimethylsulfoxid (DMSO).

Die Menge des Lösungsmittels ist nicht kritisch, vorzugsweise können 5 g bis 500 g Lösungsmittel je g des zu bildenden Produkts zugesetzt werden.

In der Regel wird bei einem Druck von 1 bis 200 bar gearbeitet, bevorzugt jedoch bei Normaldruck.

Die Reaktionstemperatur für die zuvor beschriebenen Umsetzungen liegt je nach den angewendeten Bedingungen zwischen etwa -10 und 200 °C, normalerweise zwischen -5 und 100 °C, bevorzugt zwischen 0 und 80 °C.

Die Reaktionszeit liegt je nach den angewendeten Bedingungen zwischen einigen Minuten und mehreren Tagen, vorzugsweise im Bereich von mehreren Stunden.

Die Reaktion kann auch in heterogener Phase ausgeführt werden, wobei vorzugsweise eine wässrige Phase und eine Benzol- oder Toluol-Phase verwendet werden. Hier kommt ein Phasentransfer-Katalysator zum Einsatz, wie beispielsweise Tetrabutylammoniumiodid und gegebenenfalls ein Acylierungskatalysator, wie beispielsweise Dimethylaminopyridin.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen.

Verbindungen der Formel I können ferner erhalten werden, indem man sie aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Bevorzugte Ausgangsstoffe sind auch die Oxadiazolderivate, die in die entsprechenden Amidinoverbindungen überführt werden können.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Tolyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-Iodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Ferner kann man freie Aminogruppen in üblicher Weise mit einem Säurechlorid oder -anhydrid acylieren oder mit einem unsubstituierten oder substituierten Alkylhalogenid alkylieren, oder mit CH3-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30 °C.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkyl- oder Silylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50 °C, vorzugsweise arbeitet man zwischen 15 und 30 °C (Raumtemperatur, RT).

Die Gruppen BOC, OBut und Mtr können z. B. bevorzugt mit TFA in Dichlormethan oder mit etwa 3 bis 5n HCl in Dioxan bei 15-30 °C abgespalten werden, die FMOC-Gruppe mit einer etwa 5- bis 50 %igen Lösung von Dimethylamin, Diethylamin oder Piperidin in DMF bei 15-30 °C.

Hydrogenolytisch entfernbare Schutzgruppen (z. B. CBZ, Benzyl oder die Freisetzung der Amidinogruppe aus ihrem Oxadiazolderivat) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100 °C und Drucken zwischen etwa 1 und 200 bar, bevorzugt bei 20-30 °C und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30 °C.

Ester können z.B. mit Essigsäure oder mit NaOH oder KOH in Wasser, Wasser-THF oder Wasser-Dioxan bei Temperaturen zwischen 0 und 100 °C verseift werden.

Weitere Methoden zur Entfernung von Schutzgruppen ist beispielsweise in Theodora W. Green, Peter G. M. Wuts: Protective Groups in Organic Synthesis, 3rd Edition John Wiley & Sons (1999) beschrieben.

Erfindungsgemäße Verbindungen der Formel I können aufgrund ihrer Molekülstruktur chiral sein und dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische, biochemische oder physikalische Maßnahmen, aufgetrennt oder bereits als solche bei der Synthese eingesetzt werden.

Durch übliche Aufarbeitungsschritte wie z.B. Wasserzugabe zum Reaktionsgemisch und Extraktion können die Verbindungen der Formel I nach Entfernung des Lösungsmittels erhalten werden. Es kann vorteilhaft sein, zur weiteren Reinigung des Produktes eine Destillation oder Kristallisation anzuschließen.

Ein weiterer Gegenstand der Erfindung sind Arzneimittel, enthaltend wenigstens eine erfindungsgemäße Verbindung und/oder ihre physiologisch unbedenklichen Salze, Solvate und Stereoisomere, einschließlich deren Mischungen in allen Verhältnissen.
Weiterhin kann eine erfindungsgemäße pharmazeutische Zubereitung, weitere Träger- und/oder Hilfsstoffe sowie gegebenenfalls einen oder mehrere weitere Arzneimittelwirkstoffe enthalten.

Gegenstand der Erfindung ist ferner ein Verfahren zur Herstellung eines Arzneimittels, dadurch gekennzeichnet, dass man eine erfindungsgemäße Verbindung und/oder eines ihrer physiologisch unbedenklichen Salze, Solvate und Stereoisomere, einschließlich deren Mischungen in allen Verhältnissen zusammen mit einem festen, flüssigen oder halbflüssigen Träger- oder Hilfsstoff in eine geeignete Dosierungsform bringt.

Gegenstand der Erfindung ist auch ein Set (Kit) bestehend aus getrennten Packungen von
a) einer wirksamen Menge einer erfindungsgemäßen Verbindung und/oder ihrer physiologisch unbedenklichen Salze, Solvate und Stereoisomere, einschließlich deren Mischungen in allen Verhältnissen und
b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Solvate und Stereoisomere, einschließlich deren Mischungen in allen Verhältnissen, und einer wirksamen Menge eines weiteren Arzneimittelwirkstoffs gelöst oder in lyophylisierter Form vorliegt.

Arzneimittel können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Geschlecht, Gewicht und Zustand des Patienten. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche Arzneimitel mit einem der im pharmazeutischen Fachgebiet allgemein bekannten Verfahren herstellen.

Arzneimittel lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Arzneimittel können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepasste Arzneimittel können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-Öl-Flüssigemulsionen dargereicht werden.

So lässt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmifteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch lässt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze und Solvate lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze und Solvate können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepasste Arzneimittel können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6):318, 1986 allgemein beschrieben.

An die topische Verabreichung angepasste Arzneimittel können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepassten Arzneimittel gehören Augentropfen, wobei der Wirkstoff in einem geeigneten Träger, insbesondere einem wässrigen Lösungsmittel, gelöst oder suspendiert ist.

An die topische Applikation im Mund angepasste Arzneimittel umfassen Lutschtabletten, Pastillen und Mundspülmittel.

An die rektale Verabreichung angepasste Arzneimittel können in Form von Zäpfchen oder Einläufen dargereicht werden.

An die nasale Verabreichung angepasste Arzneimittel in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste Arzneimittel umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepasste Arzneimittel können als Pessare, Tampons, Cremes, Gele, Pasten, Schäume oder Sprayformulierungen dargereicht werden.

Zu den an die parenterale Verabreichung angepassten Arzneimittel gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, dass die erfindungsgemäßen Arzneimittel neben den obigen besonders erwähnten Bestandteilen andere im Fachgebiet übliche Mittel mit Bezug auf die jeweilige Art der pharmazeutischen Formulierung enthalten können; so können beispielsweise für die orale Verabreichung geeignete Arzneimittel Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Empfängers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer Verbindung der Formel I für die Behandlung der erfindungsgemäßen Erkrankungen im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so dass die Gesamttagesdosis die gleiche ist. Eine wirksame Menge eines Salzes oder Solvats kann als Anteil der wirksamen Menge der erfindungsgemäßen Verbindung *per se* bestimmt werden.

Die erfindungsgemäßen Verbindungen zeigen eine vorteilhafte biologische Aktivität, die in Enzym-Assays leicht nachweisbar ist. In derartigen auf Enzymen basierenden Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Gegenstand der vorliegenden Erfindung sind erfindungsgemäße Verbindungen als Effektoren, bevorzugt als Inhibitoren der hier beschriebenen Signalwege. Besonders bevorzugter Gegenstand der Erfindung sind deshalb erfindungsgemäße Verbindungen als Aktivatoren und Inhibitoren von Tyrosinkinasen, bevorzugt als Inhibitoren von Rezeptor-Tyrosinkinasen, insbesondere der Insulin-Unterfamilie, zu der INS-R, IGF-IR und IR-R zählen. Hierbei zeigen die erfindungsgemäßen Verbindungen eine besondere Wirkung bei der Inhibierung der Rezeptortyrosinkinase IGF-1 R.

Wie vorstehend besprochen, sind die durch die erfindungsgemäßen Verbindungen beeinflussten Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind.

Ein weiterer Gegenstand der vorliegenden Erfindung ist deshalb die Verwendung von erfindungsgemäßen Verbindungen und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Krankheiten, insbesondere solcher Krankheiten, die durch Kinasen und/oder durch kinase-vermittelte Signaltransduktion verursacht, vermittelt und/oder propagiert werden. Bevorzugt sind hierbei Tyrosinkinasen, ausgewählt aus der Gruppe der Rezeptor-Tyrosinkinasen. Besonders bevorzugt handelt es sich dabei um IGF-1R.

Außerdem eignen sich die vorliegenden Verbindungen als pharmazeutische Wirkstoffe für Säugetiere, insbesondere für den Menschen, bei der Behandlung von tyrosinkinasebedingten Krankheiten. Der Ausdruck "tyrosinkinasebedingte Krankheiten " bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen Krebs, Tumorwachstum, Arteriosklerose, diabetischer Retinopathie und Entzündungserkrankungen.

Gewöhnlich werden die hier besprochenen Erkrankungen in zwei Gruppen eingeteilt, in hyperproliferative und nicht-hyperproliferative Erkrankungen. In diesem Zusammenhang werden Psoriasis, Arthritis, Entzündungen, Endometriose, Vernarbung, gutartige Prostatahyperplasie, immunologische Krankheiten, Autoimmunkrankheiten und Immunschwächekrankheiten als nicht-krebsartige Krankheiten angesehen, von denen Arthritis, Entzündung, immunologische Krankheiten, Autoimmunkrankheiten und Immunschwächekrankheiten gewöhnlich als nicht-hyperproliferative Erkrankungen angesehen werden.

In diesem Zusammenhang sind Hirnkrebs, Lungenkrebs, Plattenepithelkrebs, Blasenkrebs, Magenkrebs, Pankreaskrebs, Leberkrebs, Nierenkrebs, Darmkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich zur Gruppe der hyperproliferative Erkrankungen gezählt werden. Insbesondere krebsartiges Zellwachstum und insbesondere durch IGF-1 R direkt oder indirekt vermitteltes krebsartiges Zellwachstum ist eine Erkrankung, die ein Ziel der vorliegenden Erfindung darstellt.

Gegenstand der vorliegenden Erfindung ist deshalb die Verwendung von erfindungsgemäßen Verbindungen zur Herstellung eines Medikaments für die Behandlung und/oder Prophylaxe der genannten Erkrankungen sowie auch ein Verfahren zur Behandlung der genannten Erkrankungen, umfassend die Verabreichung eines oder mehrerer erfindungsgemäßer Verbindungen an einen Patienten mit Bedarf an einer derartigen Verabreichung.

Der Empfänger oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Empfänglichkeit einer bestimmten Zelle gegenüber der Behandlung mit den erfindungsgemäßen Verbindungen kann durch *in vitro*-Tests bestimmt werden. Typischerweise wird eine Kultur der Zelle mit einer erfindungsgemäßen Verbindung bei verschiedenen Konzentrationen für eine Zeitdauer inkubiert, die ausreicht, um den Wirkstoffen zu ermöglichen, Zelltod zu induzieren oder Migration zu inhibieren, gewöhnlich zwischen ungefähr einer Stunde und einer Woche. Zu *in vitro*-Tests können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der spezifischen Zellzahl und kann fortgesetzt werden, bis im Wesentlichen keine unerwünschten Zellen mehr im Körper nachgewiesen werden.

Zur Identifikation von Kinase-Inhibitoren stehen verschiedene Assay-Systeme zur Verfügung. Beim Scintillation-Proximity-Assay (Sorg et al., J. of. Biomolecular Screening: 7:11-19, 2002) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 191-214, 2002).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., Biochem. J. 366:977-981, 2002).

Es gibt viele mit einer Deregulation der Zellproliferation und des Zelltods (Apoptose) einhergehende Erkrankungen und Krankheitszustände. Die Erkrankungen und Krankheitszustände die durch erfindungsgemäße Verbindungen behandelt, verhindert oder gelindert werden können umfassen die nachfolgend aufgeführten Erkrankungen und Krankheitszustände, sind jedoch nicht darauf beschränkt. Die erfindungsgemäßen Verbindungen sind nützlich bei der Behandlung und/oder Prophylaxe einer Reihe verschiedener Erkrankungen und Krankheitszustände, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die vorliegende Erfindung umfasst die Verwendung der erfindungsgemäßen Verbindungen zur Behandlung oder Vorbeugung von Krebs. Gegenstand der Erfindung ist insbesondere die Verwendung von erfindungsgemäßen Verbindungen und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von festen Tumoren, wobei der feste Tumor besonders bevorzugt aus der Gruppe bestehend aus Gehirntumor, Tumor des Urogenitaltrakts, Tumor des lymphatischen Systems, Magentumor, Kehlkopftumor, Lungentumor ausgewählt ist. Bevorzugt können auch feste Tumore ausgewählt aus der Gruppe bestehend aus Monozytenleukämie, Lungenadenokarzinom, kleinzellige und nicht- kleinzellige Lungenkarzinome, Nierenzellkarzinom, Endometriumkarzinom, multiples Myelom, Prostatakrebs, Kolorektalkrebs, Bauchspeicheldrüsenkrebs, Glioblastome und Brustkarzinom mit Medikamenten enthaltend erfindungsgemäße Verbindungen behandelt werden.

Die erfindungsgemäßen Verbindungen können an Patienten zur Behandlung von Krebs verabreicht werden. Die vorliegenden Verbindungen hemmen über die Bindung an IGF-1 R die Tumorangiogenese und beeinflussen so das Wachstum von Tumoren (S.E. Dunn et al. Mol Carcinog. 2000 Jan;27(1):10-7). Die Eigenschaften der erfindungsgemäßen Verbindungen lassen diese auch für die Behandlung bestimmter Formen von Blindheit, die mit Retina-Gefäßneubildung in Zusammenhang stehen, geeignet erscheinen.

Gegenstand der Erfindung ist deshalb auch die Verwendung von erfindungsgemäßen Verbindungen und/oder ihrer physiologisch unbedenklichen Salze, Solvate und Stereoisomere, einschließlich deren Mischungen in allen Verhältnissen zur Herstellung eines Medikaments zur Behandlung und oder Prophylaxe von Krankheiten, die durch Angiogenese verursacht, vermittelt und/oder propagiert werden.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Gegenstand der Erfindung ist deshalb auch die Verwendung der erfindungsgemäßen Verbindungen zur Herstellung eines Medikaments zur Behandlung und/oder Prophylaxe der vorstehenden Erkrankungen.

Die Verwendung von erfindungsgemäßen Verbindungen und/oder ihrer physiologisch unbedenklichen Salze und Solvate zur Herstellung eines Medikaments zur Behandlung und/oder Prophylaxe von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.

Bevorzugt ist die Verwendung zur Behandlung von Erkrankungen, vorzugsweise aus der Gruppe der hyperproliferativen und nichthyperproliferativen Erkrankungen.
Hierbei handelt es sich um Krebserkrankungen oder nicht-krebsartige Erkrankungen.

Gegenstand der Erfindung ist auch Verwendung erfindungsgemäßer Verbindungen und/oder ihrer physiologisch unbedenklichen Salze, Solvate und Stereoisomere, einschließlich deren Mischungen in allen Verhältnissen zur Herstellung eines Medikaments zur Behandlung von Krankheiten, ausgewählt aus der Gruppe der nicht-krebsartigen Erkrankungen bestehend aus Psoriasis, Arthritis, Entzündungen, Endometriose, Vernarbung, gutartiger Prostatahyperplasie, immunologischer Krankheiten, Autoimmunkrankheiten und Immunschwächekrankheiten.

Ein weiterer Gegenstand der Erfindung ist die Verwendung von erfindungsgemäßen Verbindungen und/oder ihrer physiologisch unbedenklichen Salze, Solvate und Stereoisomere, einschließlich deren Mischungen in allen Verhältnissen zur Herstellung eines Medikaments zur Behandlung von Krankheiten, ausgewählt aus der Gruppe der krebsartigen Erkrankungen bestehend aus Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, multiplem Myelom, chronischer Leukämie und akuter Leukämie.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, zytotoxische Stoffe, antiproliferative Mittel, Prenyl-Proteintransferaseinhibitoren, HMG-CoA-Reduktase-Inhibitoren, HIV-Protease-Inhibitoren, Reverse-Transkriptase-Inhibitoren, Wachstumsfaktor-Inhibitoren sowie Angiogeneseinhibitoren. Die vorliegenden Verbindungen eignen sich insbesondere zur gemeinsamen Anwendung mit Radiotherapie.
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Ostrogenrezeptormodulatoren zählen zum Beispiel Tamoxifen, Raloxifen, ldoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, wobei diese Aufzählung keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Inhibitoren, Nilutamid, Flutamid, Bicalutamid, Liarozol und Abirateronacetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"zytotoxische Stoffe" bezieht sich auf Verbindungen, die in erster Linie durch direkte Einwirkung auf die Zellfunktion zum Zelltod führen oder die die Zellmitose hemmen oder diese stören, darunter Alkylierungsmittel, Tumornekrosefaktoren, interkaliernde Mittel, Mikrotubulin-Inhibitoren und Topoisomerase-Inhibitoren.
Zu den zytotoxischen Stoffen zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(11)]bis[diamin(chlor)platin(11)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Inhibitoren zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Inhibitoren sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Amino-propylamino)-7,10-dihydroxy-2-(2-hydroxyethylaminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethylamino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diaza-tetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch monoklonale Antikörper gegen Wachstumsfaktoren wie Erbitux, Trastuzumab, sowie Tumorsuppressorgene, wie p53, die über rekombinanten virusvermittelten Gentransfer abgegeben werden können (siehe z.B. US-Patent Nr. 6,069,134).

### Ausführungsbeispiele

### Beispiel A1: Herstellung von 3,4,5Trimethoxy-4-(1H-pyrrolo[2,3-b]pyridin-2-yl)-phenol

Unter Stickstoff werden bei 0 °C 33 mL Lithium-Diisopropylamid Lösung (1 M in THF) vorgelegt und unter Rühren bei 0-5 °C eine Lösung aus 3,6 g 3-Methylpyridin 1 in 50 mL THF zugetropft. Bei der angegebenen Temperatur wird 30 Minuten nachgerührt und anschließend eine Lösung von 5 g 3,4,5-Trimethoxybenzonitril in 50 mLTHF zugegeben. Man rührt 1,5 h bei 0-5 °C nach und gibt schließlich weitere 33 mL Lithium-Diisopropylamid Lösung zu. Anschließend wird das Reaktionsgemisch 2 h auf 80 °C erwärmt. Zur Aufarbeitung läßt man den Ansatz auf Raumtemperatur abkühlen und gießt die Mischung auf Eis. Nach der Phasentrennung wird noch dreimal mit je 100 mL Dichlormethan extrahiert, die vereinigten organischen Phasen getrocknet und das Lösungsmittel im Vakuum entfernt. Der Rückstand wird mit Ethylacetat über eine Kieselgelsäule chromatographisch aufgereinigt. Man erhält 4,3 g gelber Kristalle, die einen Schmelzpunkt von 174,0 - 175,5 °C zeigen.

Weitere Verbindungen der Formel VII, die auf diese Weise hergestellt werden können sind z.B.:
- 2,6-Dimethoxy-4-(1H-pyrrolo[2,3-b]pyridin-2-yl)-phenol und
- 2-(1H-Indol-5-yl)-1H-pyrrolo[2,3-b]pyridin

### Beispiel A2: Herstellung von 4-Chloro-2-(3,4,5-trimethoxy-phenyl)-1H-pyrrolo[2, 3-b]pyridin

Zu einer Lösung aus 1 g des nach Beispiel 1 hergestellten 7-Azaindols in 30 mL redestilliertem 1,2-Dimethoxyethan werden 1,5 g 3-Chlorperbenzoesäure gegeben und 1,5 h bei Raumtemperatur nachgerührt. Schließlich gibt man 40 mL Diethylether zu und lässt 1,5 h bei Raumtemperatur nachrühren. Die entstehenden Kristalle werden abgesaugt, mit Ether gewaschen und an der Luft getrocknet. Man erhält 0,9 g (56%) gelbe Kristalle.
Eine Suspension aus 0,9 g des 3-Chlorperbenzoates wird in 10 mL Wasser gelöst und mit einer gesättigten Kaliumcarbonat-Lösung zunächst auf pH = 9 dann auf pH = 12 eingestellt und für 12 h bei Raumtemperatur nachgerührt, wobei Kristalle ausfallen. Die Kristalle werden abgesaugt, mit Wasser gewaschen und bei 80 °C 3 h im Vakuum getrocknet. Man erhält 0,5 g (85%) beige Kristalle.
500 mg des N-Oxids werden zusammen mit 10 ml POCl₃ 2h bei 110 °C erhitzt. Nach dem Abkühlen der Reaktionsmischung wird diese auf Eiswasser gegossen und mit konzentrierter Natronlauge auf pH = 13 gestellt. Der resultierende Niederschlag wird mit Ethylacetat verrührt, über Kieselgur abgesaugt und der Rückstand verworfen. Vom Filtrat wird die organische Phase abgetrennt, getrocknet und das Lösungsmittel im Vakuum entfernt. Der Rückstand wird mit Ethylacetat chromatographiert und die Pröduktfraktionen aus Ethylacetat kristallisiert. Man erhält 0,4 g (75 %) gelbe Kristalle mit einem Schmelzpunkt von 188,0 - 190,0°C.

Eine weitere Verbindung der Formel VI, die auf diese Weise hergestellt werden kann ist z.B. 4-Chloro-2-(3-methoxy-phenyl)-1H-pyrrolo[2,3-b]pyridin

### Beispiel A3: Herstellung von Chinolin-3-yl-[2-(3,4,5-trimethoxy-phenyl)-1H-pyrrolo[2,3b]pyridin-4-yl]-amin

Man löst 2 g des nach Beispiel 2 hergestellten Verbindung der Formel VI in 50 mL Dioxan, gibt 1,1 g Kalium-tert.-Butylat hinzu und erwärmt auf 80 °C. Dann fügt man 20 mg 2-(Dimethylamino)ferrocen-1-yl-palladium(II)chlorid Dinorbornyl-phosphin-Komplex hinzu und schließlich 1,15 g 3-Aminochinolin. Nach 12 h lässt man den Ansatz auf Raumtemperatur abkühlen und verteilt das Reaktionsgemisch zwischen Ethylacetat und Wasser. Die organische Phase wird getrocknet, im Vakuum vom Lösungsmittel befreit und an Kieselgel chromatographiert. Man erhält 1,1 g gelbe Kristalle (Schmelpunkt 279,5 - 280 °C). 300 mg des so hergestellten Produktes werden in 20 mL Aceton und 20 mL Methanol gelöst und der pH Wert der Lösung mit ethanolischer Salzsäure auf 3 eingestellt. Die ausfallenden Kristalle werden abgesaugt mit Diethylether gewaschen und an der Luft getrocknet. Man erhält 300 mg organgefarbene Kristalle. Schmelzpunkt: 227,0 - 228,5 °C

| Elementaranalyse | | C | H | Cl | N |
|---|---|---|---|---|---|
| | Gesucht: | 58,0 | 5,1 | 13,7 | 10,8 |
| | Gefunden: | 57,5 | 5,3 | 13,2 | 11,0 |
| | (berechnet auf Dihydrochlorid Hydrat | | | | |

Weitere Verbindungen der Formel I, die auf diese Weise hergestellt werden können sind z.B.:
- 3-[2-(3,4,5-Trimethoxy-phenyl)-1H-pyrrolo[2,3-b]pyridin-4-ylamino]-benzonitril
- (2-Pyridin-2-yl-ethyl)-[2-(3,4,5-trimethoxy-phenyl)-1H-pyrrolo[2,3-b]pyridin-4-yl]-amin
- (2-Pyridin-3-yl-ethyl)-[2-(3,4,5-trimethoxy-phenyl)-1H-pyrrolo[2,3-b]pyridin-4-yl]-amin
- Pyridin-3-ylmethyl-[2-(3,4,5-trimethoxy-phenyl)-1H-pyrrolo[2,3-b]pyridin-4-yl]-amin
- Pyrimidin-2-yl-[2-(3,4,5-trimethoxy-phenyl)-1H-pyrrolo[2,3-b]pyridin-4-yl]-amin
- (3-Chloro-4-fluoro-phenyl)-[2-(3,4,5-trimethoxy-phenyl)-1H-pyrrolo[2,3-b]pyridin-4-yl]-amin
- (3-Fluoro-phenyl)-[2-(3,4,5-trimethoxy-phenyl)-1H-pyrrolo[2,3-b]pyridin-4-yl]-amin
- [4-Methoxy-3-(4-methyl-piperazin-1-yl)-phenyl]-[2-(3,4,5-trimethoxyphenyl)-1H-pyrrolo[2,3-b]pyridin-4-yl]-amin
- Pyridin-3-yl-[2-(3,4,5-trimethoxy-phenyl)-1H-pyrrolo[2,3-b]pyridin-4-yl]-amin

### Beispiel B1: Herstellung von 2-Chloro-3-iodo-pyridin-4-yl-amin

Kommerziell erhältliches 2-Chlor-4-aminopyridin (15 g, 0,1 mol) und 37,2 g (0,4 mol) Natriumcarbonat werden in 200 ml Wasser suspendiert und auf 100°C erwärmt. Zu der entstandenen Lösung gibt man 58,3 g (0,4 mol) Kaliumiodid und 59,4 g (0,2 mol) lod und rührt bei der angegebenen Temperatur für 12 h. Anschließend wird mit Natronlauge auf pH 13 eingestellt, mit Natriumthiosulfat bis zur vollständigen Entfärbung behandelt und mit Essigester extrahiert. Nach chromatographischer Aufreinigung erhält man 5 g (17%) Feststoff.

### Beispiel B2: Herstellung von 4-Chloro-3-(3-methoxy-phenyl)-2-trimethylsilanyl-1H-pyrrolo[3,2-c]pyridin

7,6 g (30 mmol) 2-Chloro-3-iodo-pyridin-4-yl-amin aus Beispiel B1, 1,7 g (41 mmol) Lithiumchlorid und 15,9 g (120 mmol) Natriumcarbonat werden in 100 ml DMF gelöst und unter Stickstoff bei 100°C mit 8 g (39 mmol) kommerziellem (3-Methoxyphenyl)-ethinyl-trimethylsilan und 4,9 g (6 mmol) kommerziellem Pd(dppf)₂Cl₂ * H₂Cl₂ versetzt. Die Mischung wird für 12 h bei der angegebenen Temperatur gerührt und anschließend bei Raumtemperatur (RT) auf Wasser gegossen und mit Essigester extrahiert. Die organische Phase wird eingeengt und über Kieselgel chromatographisch aufgereinigt. Die vereinigten Produktfraktionen (5,3 g; 53%; hellbraunes Öl) werden für die Folgereaktion verwendet.

### Beispiel B3: Herstellung von (4-Methoxy-benzyl)-[3-(3-methoxy-phenyl)-1H-pyrrolo[3,2-c]pyridin-4-yl]-amin

3 g (9 mmol) 4-Chloro-3-(3-methoxy-phenyl)-2-trimethylsilanyl-1H-pyrrolo[3,2-c]pyridin aus Beispiel B2, 1,8 g (13.5 mmol) 4-Methoxybenzylamin und 2,2 g (20 mmol) Kalium tert.-butylat werden in 50 ml 1,4-Dioxan suspendiert und bei 100°C mit 15 mg (0,02 mmol) 2-(Dimethylamino)ferrocen-1-yl-palladium(II)chlorid Dinorbornyl-phosphin Komplex versetzt. Nach 12 h wird der Ansatz bei RT mit 1 N Natronlauge auf pH 13 gebracht und mit Essigester extrahiert. Die organische Phase wird an Kieselgel aufgereinigt, die vereinigten Produktfraktionen werden mit Aceton und ethanolischer Salzsäure umkristallisiert. Man erhält 500 mg (14%) des entsprechenden Hydrochlorids.

### Beispiel B4: Herstellung von {4-[4-(4-Methoxy-benzylamino)-3-(3-methoxy-phenyl)-pyrrolo[3,2-c]pyridin-1-yl]-butyl}carbamidsäure tert.-Butylester

450 mg (1,1 mmol) (4-Methoxy-benzyl)-[3-(3-methoxy-phenyl)-1H-pyrrolo[3,2-c]pyridine-4-yl]-amin aus Beispiel B3, 428 mg (1,7 mmol) 4-(BOC-amino)-butylbromid und 0,9 g (2,8 mmol) Cäsiumcarbonat werden in 60 ml DMF über 12 h auf 60°C erwärmt. Nach wässriger Aufarbeitung bei RT und chromatographischer Aufreinigung erhält man 600 mg (82%) eines farblosen Öls.

### Beispiel C: Hemmung von IGF-1 R (IC₅₀)

Kultivierte humane Tumorzellen, die den IGF1-Rezeptor (IGF1R) exprimieren (z.B. MCF-7 oder Calu-6), werden mit humanem IGF1, dem natürlichen Liganden des IGF1R stimuliert. Die Stimulation induziert eine Autophosphorylierung von Tyrosinresten in der cytoplasmatischen IGF1 R-Domäne, welche Signaltransduktionskaskaden auslöst, die zur Apoptosehemmung und Proliferation der Zellen führen.
Die Menge an phosphoryliertem IGF1R wird durch einen rezeptorspezifischen Capture-ELISA oder einen analogen LUMINEX-Assay bestimmt. Der IGF1R aus Zelllysaten wird mittels eines spezifischen Antikörpers an eine 96-well ELISA-Platte bzw. LUMINEX-Beads gebunden ("Capturing"), und die Tyrosinphosphorylierung mit einem Biotin-markierten anti-Phosphotyrosin Antikörper und einem Streptavidin-Peroxidase-Konjugat durch ein Chemilumineszenz-Verfahren bzw. mittels eine Fluoreszenzmarkierten anti-Phosphotyrosin-Antikörpers detektiert.
Zur Bestimmung der Aktivität von Kinaseinhibitoren werden Zellen mit ansteigenden Konzentrationen dieser Verbindungen für 45 min vorbehandelt und anschließend für 5 min mit IGF1 stimuliert. Als interne Kontrolle wird die biologische Aktivität des Liganden IGF1 überprüft sowie eine Konzentrationsreihe eines IGF1R-Referenzinhibitors vermessen.

Für Chinolin-3-yl-[2-(3,4,5-trimethoxy-phenyl)-1H-pyrrolo[2,3b]pyridin-4-yl]-amin wird nach dieser Vorschrift folgendes Ergebnis erhalten:
Die Substanz hemmt die Kinase IGF-1R zu 50%, wenn die Verbindung in einer Konzentration von 14 µM vorliegt.

Weitere Inhibitionskonstanten von erfindungsgemäßen Verbindungen sind in der Tabelle 1 aufgeführt.

Die nachfolgenden Beispiele betreffen pharmazeutische Zubereitungen:

### Beispiel D1: Injektionsgläser

Eine Lösung von 100 g eines erfindungsgemäßen Wirkstoffes und 5 g Dinatriumhydrogenphosphat wird in 3 L zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel D2: Suppositorien

Man schmilzt ein Gemisch von 20 g eines erfindungsgemäßen Wirkstoffes mit 100 g Sojalecithin und 1400 g Kakaobutter, gießt in Formen und lässt erkalten. Jedes Suppositorium enthält 20 mg Wirkstoff.

### Beispiel D3: Lösung

Man bereitet eine Lösung aus 1 g eines erfindungsgemäßen Wirkstoffes, 9,38 g NaH₂PO4 · 2 H₂O, 28,48 g Na₂HPO₄ · 12 H₂O und 0,1 g Benzalkoniumchlorid in 940 mL zweifach destilliertem Wasser. Man stellt auf pH 6,8 ein, füllt auf 1 L auf und sterilisiert durch Bestrahlung. Diese Lösung kann in Form von Augentropfen verwendet werden.

### Beispiel D4: Salbe

Man mischt 500 mg eines erfindungsgemäßen Wirkstoffes mit 99,5 g Vaseline unter aseptischen Bedingungen.

### Beispiel D5: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Wirkstoff enthält.

### Beispiel D6: Dragees

Analog Beispiel 5e werden Tabletten gepresst, die anschließend in üblicher Weise mit einem Überzug aus Saccharose, Kartoffelstärke, Talk, Tragant und Farbstoff überzogen werden.

### Beispiel D7: Kapseln

2 kg Wirkstoff werden in üblicher Weise in Hartgelatine kapseln gefüllt, so dass jede Kapsel 20 mg des Wirkstoffs enthält.

### Beispiel D8: Ampullen

Eine Lösung von 1 kg eines erfindungsgemäßen Wirkstoffes in 60 L zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I, 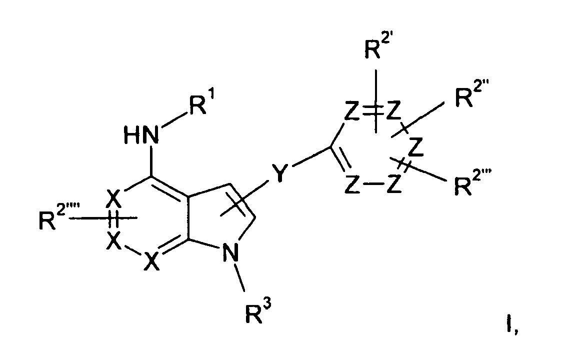 worin
R¹ H, A, Ar, Ar-A oder A-Ar,
A unverzweigtes, verzweigtes oder cyclisches Alkyl mit 1-14 C- Atomen, worin eine oder zwei CH₂-Gruppen durch ein O- o- der S-Atom und/oder durch eine NH, NA, CONH, NHCO oder -CH=CH- Gruppe und/oder auch 1-7 H-Atome durch Hal er- setzt sein können, und worin eine oder zwei CH₃-Gruppen durch NH₂, NAH, NA₂, NHCOOA, NHCONHA, NHCONHAr, oder CN ersetzt sein können,
Ar einen ein- oder zweikernigen aromatischen Homo- oder He- terocyclus mit 1 bis 4 N-, O- und/oder S-Atomen und 5 bis 10 Gerüstatomen, der unsubstituiert oder ein-, zwei- oder drei- fach durch Carbonylsauerstoff, Hal, A, OH, OA, NH₂, NHA, NA₂, NO₂, CN, OCN, SCN, COOH, COOA, CONH₂, CONHA, CONA₂, NHCOA, NHCONH₂, NHSO₂A, CHO, COA, SO₂NH₂ und/oder S(O)gA substituiert sein kann,
Ar-A Aryl-alkyl
A-Ar Alkyl-aryl
Hal F, Cl, Br oder I,
X CH oder N, wobei in jeder Verbindung der Formel I eine Gruppierung X N und zwei Gruppierungen X CH sind,
Y CH₂ oder eine gesättigte Bindung,
Z CH oder N, wobei in jeder Verbindung der Formel I höchs- tens zwei Gruppierungen Z NH und vorzugsweise eine oder keine Gruppierung Z NH sind,
R^{2'}, R^{2"}, R^{2"'}, R^{2""} jeweils unabhängig voneinander H, Hal, OH, CN, NH₂, unverzweigtes oder verzweigtes Alkyl mit 1-4, 5 oder 6 C- Atomen, worin eine CH₂-Gruppe durch ein O oder S-Atom und/oder durch eine NH, NA, CONH oder -CH=CH- Gruppe und/oder auch 1-4 H-Atome durch Hal ersetzt sein können, und worin eine CH₃-Gruppe durch NH₂, NAH, NA₂, CN oder Ar ersetzt sein kann,
R³ H, A oder Ar-A,
g 0, 1 oder 2 und
-̅ -̅ -̅ -̅ -̅ eine Einfach- oder Doppelbindung
bedeuten,
3-(Aminophenyl)-1*H*-pyrrolo[3,2-c]pyridin-4-amin nicht umfasst ist, sowie ihre pharmazeutisch unbedenklichen Salze, Solvate und Stereoisomere, einschließlich deren Mischungen in allen Verhältnissen.

2. Verbindungen nach Anspruch 1, die der Formel All entsprechen 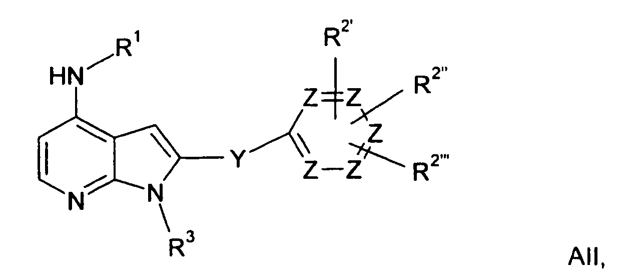 worin R¹, R², R³, Y und Z die für die Formel I gemäß Anspruch 1 angegebene Bedeutung haben sowie ihre pharmazeutisch unbedenklichen Salze, Solvate und Stereoisomere, einschließlich deren Mischungen in allen Verhältnissen.

3. Verbindungen nach Anspruch 1 oder 2, die der Formel AIII entsprechen 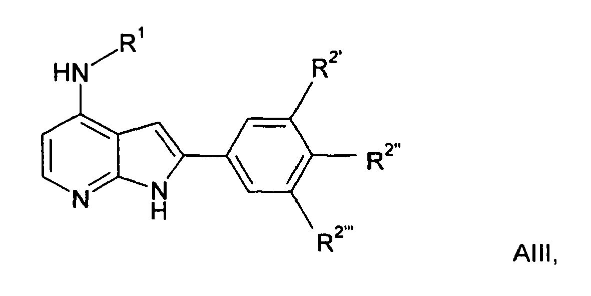 worin Y eine Bindung, Z CH, R³ H und R¹ bzw. R² die für die Formel I gemäß Anspruch 1 angegebene Bedeutung haben,
sowie ihre pharmazeutisch unbedenklichen Salze, Solvate und Stereoisomere, einschließlich deren Mischungen in allen Verhältnissen.

4. Verbindungen nach einem oder mehreren der Ansprüche 1 bis 3, worin die nicht näher bezeichneten Reste die bei der Formel I gemäß Anspruch 1 angegebene Bedeutung haben, worin jedoch
bei der Teilformel Aa
R¹ unsubstituiertes oder ein- oder mehrfach durch Hal, Cyano, Methyl oder Methoxy substituiertes Phenyl, Phenylmethyl, Pyridyl, Pyridyl methyl, Pyridylethyl, Pyrimidyl, Piperazyl, Chinoliny, Imidazoyl, Imidazyolpropyl, Pyrrolyl, Pyrrolylethyl, weiterhin H, N,N'- Dimethylaminopropyl oder Cyanobutyl oder einen der folgenden Reste 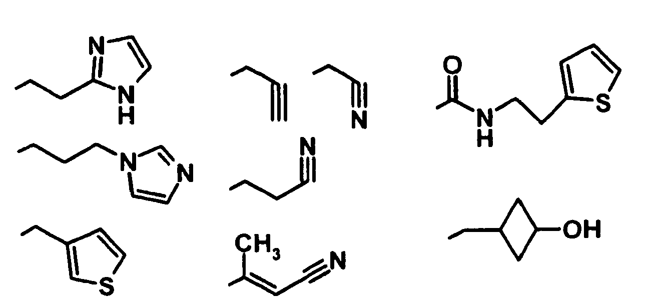 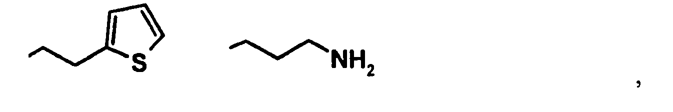 wobei die Verknüpfung mit dem Grundkörper der Formel I, All bzw. AII jeweils über die nach links stehende Bindung, die keine Methylguppe ist, erfolgt,
bedeutet,
bei der Teilformel Ab
R^{2'}, R^{2"}, R^{2'"} jeweils unabhängig voneinander H, Methoxy, Ethoxy, n- Propoxy oder i-Propoxy bedeuten,
bei der Teilformel Ac
R^{2'}, R^{2"}, R^{2'"} jeweils unabhängig voneinander H oder Methoxy bedeuten,
bei der Teilformel Ad
R^{2'}, R^{2"}, R^{2'"} jeweils Methoxy bedeuten,
bei der Teilformel Ae
R^{2'}, R^{2"}, R^{2"'} jeweils unabhängig voneinander H, Methoxy, Ethoxy, n- Propoxy oder i-Propoxy bedeuten
und R¹ die für die Teilformel Aa angegebene Bedeutung hat,
bei der Teilformel Af
R^{2'}, R^{2"}, R^{2'"} jeweils unabhängig voneinander H oder Methoxy bedeuten
und R¹ die für die Teilformel Aa angegebene Bedeutung hat,
bei der Teilformel Ag
R^{2'}, R^{2"}, R^{2'"} jeweils Methoxy bedeuten
und R¹ die für die Teilformel Aa angegebene Bedeutung hat,
sowie ihre pharmazeutisch unbedenklichen Salze, Solvate und Stereoisomere, einschließlich deren Mischungen in allen Verhältnissen.

5. Verbindungen nach Anspruch 1, die der Formel BII entsprechen 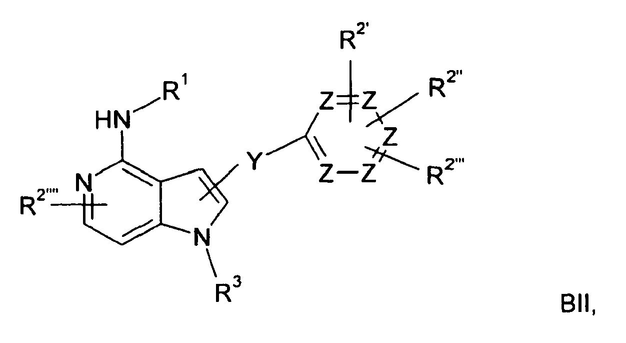 worin R¹, R², R³, Y und Z die für die Formel I gemäß Anspruch 1 angegebene Bedeutung haben sowie ihre pharmazeutisch unbedenklichen Salze, Solvate und Stereoisomere, einschließlich deren Mischungen in allen Verhältnissen.

6. Verbindungen nach Anspruch 1 oder 5, die der Formel BIII entsprechen 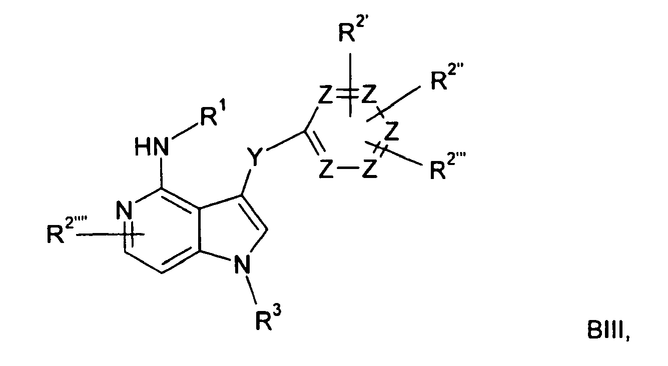 worin sämtliche Reste die für die Formel I gemäß Anspruch 1 angegebene Bedeutung haben,
sowie ihre pharmazeutisch unbedenklichen Salze, Solvate und Stereoisomere, einschließlich deren Mischungen in allen Verhältnissen.

7. Verbindungen nach einem oder mehreren der Ansprüche 1, 5 und 6, worin die nicht näher bezeichneten Reste die bei der Formel I angegebene Bedeutung haben, worin jedoch
bei der Teilformel Ba
R¹ unsubstituiertes oder ein- oder mehrfach durch Hal, Cyano, Methyl oder Methoxy substituiertes Phenyl, Phenylmethyl, Pyridyl, Pyridyl methyl, Pyridylethyl, Pyrimidyl, Piperazyl, Chinoliny, Imidazoyl, Imi dazyolpropyl, Pyrrolyl, Pyrrolylethyl sowie H bedeutet
und R² bzw. R³ die für die Formel I angegebene Bedeutung haben,
bei der Teilformel Bb
R^{2'}, R^{2"}, R^{2"'} jeweils unabhängig voneinander H, Methoxy, Ethoxy, n- Propoxy, i-Propoxy, Phenyl-methoxy oder Phenyl-ethoxy bedeuten
R^{2""} H, Hal oder NH₂
und R¹ bzw. R³ die für die Formel I angegebene Bedeutung haben,
bei der Teilformel Bc
Einer der Reste R^{2'}, R^{2"}, R^{2'"} Methoxy oder Phenyl-methoxy und die anderen beiden H bedeuten
und R¹ bzw. R³ die für die Formel I angegebene Bedeutung haben,
bei der Teilformel Bd
R³ 2-Aminoethyl, 3-Aminopropyl, 4-Aminobutyl, 5-Aminopentyl, 3- Aminomethyl-cyclobutyl, (Isoindol-1,3-dion)2-yl oder 4-(Carbamid säure-tert.-butylester)but-1-yl bedeutet
und R¹ bzw. R² die für die Formel I angegebene Bedeutung haben,
bei der Teilformel Be
R^{2'}, R^{2"}, R^{2'"} jeweils unabhängig voneinander H, Methoxy, Ethoxy, n- Propoxy, i-Propoxy, Phenyl-methoxy oder Phenyl-ethoxy,
R^{2""} H, Cl oder NH₂,
R¹ unsubstituiertes oder ein- oder mehrfach durch Hal, Cyano, Methyl oder Methoxy substituiertes Phenyl, Phenylmethyl, Pyridyl, Pyridylmethyl, Pyridylethyl, Pyrimidyl, Piperazyl, Chinoliny, Imidazoyl, Imidazyolpropyl, Pyrrolyl, Pyrrolylethyl sowie H und
R³ 2-Aminoethyl, 3-Aminopropyl, 4-Aminobutyl, 5-Aminopentyl, 3-Aminomethyl-cyclobutyl, (Isoindol-1,3-dion)2-yl oder 4-(Car bamidsäure-tert.- butylester)but-1-yl bedeutet,
bei der Teilformel Bf
einer der Reste R^{2'}, R^{2"}, R^{2'"} Methoxy oder Phenyl-methoxy und die anderen beiden H bedeuten
R¹ H, Pyridyl, Pyridylmethyl oder (4-Methoxy-phenyl)methyl und
R³ 2-Aminoethyl, 3-Aminopropyl, 4-Aminobutyl, 5-Aminopentyl, 3-Aminomethyl-cyclobutyl, (Isoindol-1,3-dion)2-yl oder 4-(Car bamidsäure-tert.- butylester)but-1-yl bedeutet,
bei der Teilformel Bg
einer der Reste R^{2'}, R^{2"}, R^{2'"} Methoxy oder Phenyl-methoxy und die anderen beiden H bedeuten
R¹ H, Pyrid-2 oder 3-yl, Pyrid-2 oder 3-yl-methyl oder (4- Methoxy-phenyl)methyl und
R³ 2-Aminoethyl, 3-Amino-n-propyl, 4-Amino-n-butyl, 5-Amino-n- pentyl, 3-Aminomethyl-cyclobut-1-yl, (Isoindol-1,3-dion)2-yl oder 4-(Carbamidsäure-tert.- butylester)but-1-yl bedeutet
sowie ihre pharmazeutisch unbedenklichen Salze, Solvate und Stereoisomere, einschließlich deren Mischungen in allen Verhältnissen.

8. Verfahren zur Herstellung von Verbindungen der Formel I sowie ihrer physiologisch unbedenklichen Salze, Solvate und Stereoisomere, **dadurch gekennzeichnet,**
**dass** man eine Verbindung der Formel (ix) 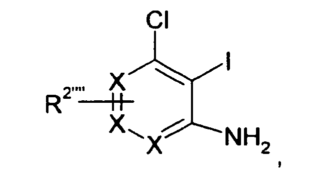 worin X die bei der Formel I angegebene Bedeutung hat mit einer Verbindung der Formel (viii), 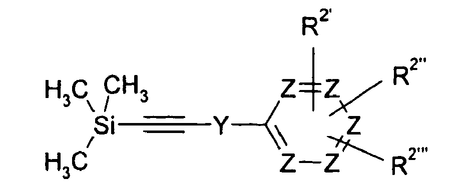 worin Z, Y und R² die bei der Formel I angegebenen Bedeutungen haben, zu einer Verbindung der Formel (vii) umsetzt, 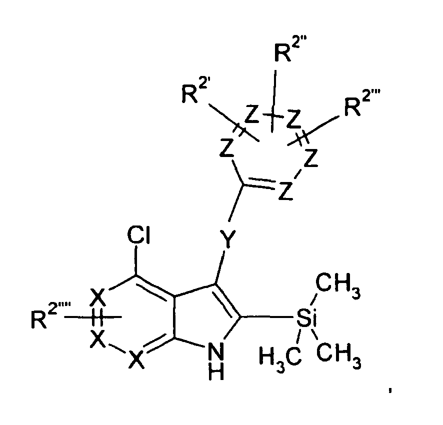 die ggf. zu einer entsprechenden 2,3-Dihydro-Indolverbindung reduziert wird, die in einem nächsten Schritt zu einer Verbindung der Formel (vi) 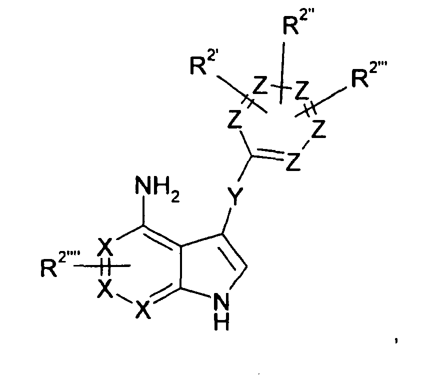 umgewandelt wird, an die, falls gewünscht, noch ein Rest R¹ und/oder ein Rest R³ unter Erhalt einer Verbindung der Formel (iii) 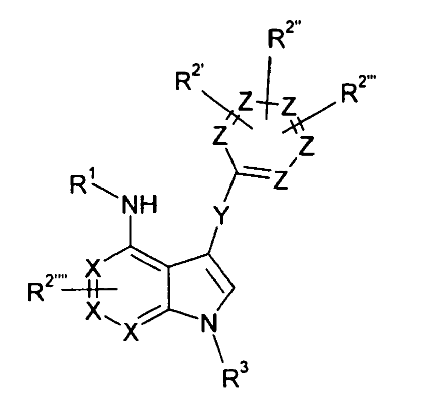 angefügt wird und ggf. eine Base oder Säure der Formel (iii) in eines ihrer Salze umwandelt,
wobei, falls die Verknüpfung des monozyklischen Arylkörpers mit dem bizyklischen Arylkörper über dessen 2-Position zu einer Verbindung der Formel (ii) 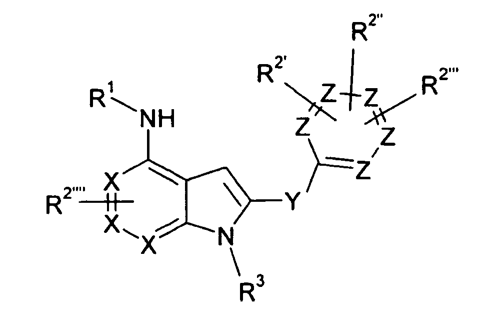 gewünscht wird, im ersten Schritt eine Verbindung der Formel (viii) ohne Silylgruppe eingesetzt wird.

9. Verbindungen nach einem oder mehreren der Ansprüche 1 bis 7 und/oder ihre physiologisch unbedenklichen Salze, Solvate und Stereoisomere, einschließlich deren Mischungen in allen Verhältnissen als Arzneimittel.

10. Arzneimittel, enthaltend wenigstens eine Verbindung nach einem oder mehreren der Ansprüche 1 bis 7 und/oder ihre physiologisch unbedenklichen Salze, Solvate und Stereoisomere, einschließlich deren Mischungen in allen Verhältnissen, sowie gegebenenfalls Träger- und/oder Hilfsstoffe.

11. Arzneimittel, enthaltend wenigstens eine Verbindung nach einem oder mehreren der Ansprüche 1 bis 7 und/oder ihre physiologisch unbedenklichen Salze, Solvate und Stereoisomere, einschließlich deren Mischungen in allen Verhältnissen sowie wenigstens einen weiteren Arzneimittelwirkstoff.

12. Set (Kit) bestehend aus getrennten Packungen von
a) einer wirksamen Menge einer Verbindung nach einem oder mehreren der Ansprüche 1 bis 7 und/oder ihrer physiologisch unbedenklichen Salze, Solvate und Stereoisomere, einschließlich deren Mischungen in allen Verhältnissen und
b) einer wirksamen Menge eines weiteren Arzneimittelwirkstoffs.

13. Verbindungen nach einem oder mehreren der Ansprüche 1 bis 7 sowie ihre physiologisch unbedenklichen Salze, Solvate und Stereoisomere, einschließlich deren Mischungen in allen Verhältnissen als Aktivatoren oder Inhibitoren von Kinasen, insbesondere Tyronsin-Kinasen.

14. Verbindungen nach einem oder mehreren der Ansprüche 1 bis 7 sowie ihre physiologisch unbedenklichen Salze, Solvate und Stereoisomere, einschließlich deren Mischungen in allen Verhältnissen als Inhibitoren der Rezeptor-Tyrosinkinase IGF-1 R.

15. Verwendung von Verbindungen nach einem oder mehreren der Ansprüche 1 bis 7 und/oder ihrer physiologisch unbedenklichen Salze, Solvate und Stereoisomere, einschließlich deren Mischungen in allen Verhältnissen zur Herstellung eines Medikaments zur Prophylaxe oder Behandlung von Krankheiten, bei denen die Hemmung der Rezeptor-Tyrosinkinase IGF-1 R zur Verbesserung des Krankheitsbildes führt.

16. Verwendung von Verbindungen nach einem oder mehreren der Ansprüche 1 bis 7 und/oder ihrer physiologisch unbedenklichen Salze, Solvate und Stereoisomere, einschließlich deren Mischungen in allen Verhältnissen zur Herstellung eines Medikaments zur Prophylaxe oder Behandlung von Krebs, Tumorwachstum, Tumorangiogenese, Arteriosklerose, der diabetischen Retinopathie und Entzündungserkrankungen.

17. Verwendung von Verbindungen nach einem oder mehreren der Ansprüche 1 bis 7 und/oder ihrer physiologisch unbedenklichen Salze, Solvate und Stereoisomere, einschließlich deren Mischungen in allen Verhältnissen zur Herstellung eines Medikaments zur Prophylaxe oder Behandlung von Brustkrebs, Prostatakrebs, Kolorektalkrebs, kleinzelligem Lungenkrebs, nicht-kleinzelligem Lungenkrebs, multiplem Myelom sowie dem Nierenzellkarzinom und dem Endometriumkarzinom.

## Claims

1. Compounds of the formula I, 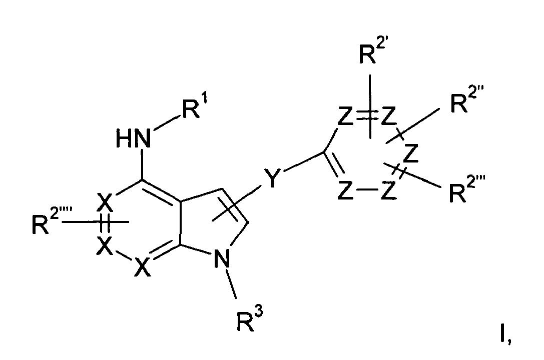 in which
R¹ denotes H, A, Ar, Ar-A or A-Ar,
A denotes unbranched, branched or cyclic alkyl having 1-14 C atoms, in which one or two CH₂ groups may be replaced by an O or S atom and/or by an NH, NA, CONH, NHCO or -CH=CH- group and/or, in addition, 1-7 H atoms may be re- placed by Hal, and in which one or two CH₃ groups may be replaced by NH₂, NAH, NA₂, NHCOOA, NHCONHA, NHCONHAr or CN,
Ar denotes a mono- or bicyclic aromatic homo- or heterocycle having 1 to 4 N, O and/or S atoms and 5 to 10 skeleton atoms, which may be unsubstituted or mono-, di- or trisubsti- tuted by carbonyl oxygen, Hal, A, OH, OA, NH₂, NHA, NA₂, NO₂, CN, OCN, SCN, COOH, COOA, CONH₂, CONHA, CONA₂, NHCOA, NHCONH₂, NHSO₂A, CHO, COA, SO₂NH₂ and/or S(O)gA,
Ar-A denotes aryl-alkyl,
A-Ar denotes alkyl-aryl,
Hal denotes F, Cl, Br or I,
X denotes CH or N, where one group X in each compound of the formula I is N and two groups X are CH,
Y denotes CH₂ or a saturated bond,
Z denotes CH or N, where at most two groups Z in each com- pound of the formula I are NH and preferably one or no group Z is NH,
R^{2'}, R^{2"}, R^{2"'}, R^{2""} each, independently of one another, denote H, Hal, OH, CN, NH₂, unbranched or branched alkyl having 1-4, 5 or 6 C atoms, in which one CH₂ group may be replaced by an O or S atom and/or by an NH, NA, CONH or -CH=CH- group and/or, in addition, 1-4 H atoms may be replaced by Hal, and in which one CH₃ group may be replaced by NH₂, NAH, NA₂, CN or Ar,
R³ denotes H, A or Ar-A,
g denotes 0, 1 or 2 and
-̅ -̅ -̅ -̅ -̅ denotes a single or double bond,
3-(aminophenyl)-1*H*-pyrrolo[3,2-c]pyridin-4-amine is not included, and pharmaceutically acceptable salts, solvates and stereoisomers thereof, including mixtures thereof in all ratios.

2. Compounds according to Claim 1 which conform to the formula AIII 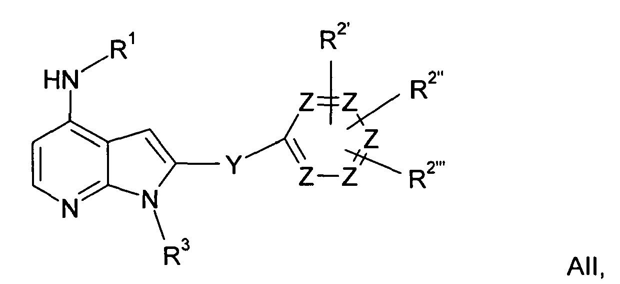 in which R¹, R², R³, Y and Z have the meanings indicated for the formula I according to Claim 1, and pharmaceutically acceptable salts, solvates and stereoisomers thereof, including mixtures thereof in all ratios.

3. Compounds according to Claim 1 or 2 which conform to the formula AIII 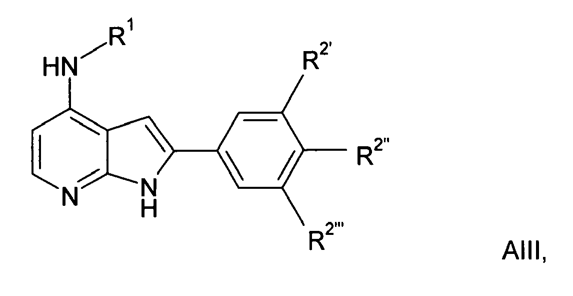 in which Y denotes a bond, Z denotes CH, R³ denotes H, and R¹ and R² have the meaning indicated for the formula I according to Claim 1,
and pharmaceutically acceptable salts, solvates and stereoisomers thereof, including mixtures thereof in all ratios.

4. Compounds according to one or more of Claims 1 to 3, in which the radicals not designated in greater detail have the meaning indicated for the formula I according to Claim 1, but in which
in the sub-formula Aa
R¹ denotes phenyl, phenylmethyl, pyridyl, pyridylmethyl, pyridylethyl, pyrimidyl, piperazyl, quinolinyl, imidazoyl, imidazoylpropyl, pyrrolyl, pyrrolylethyl, each of which is unsubstituted or mono- or polysub- stituted by Hal, cyano, methyl or methoxy, furthermore H, N,N'- dimethylaminopropyl or cyanobutyl or one of the following radicals: 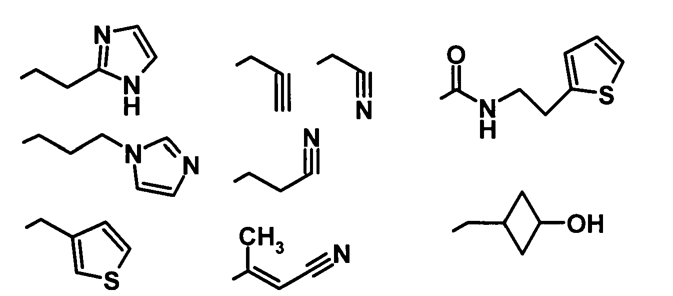 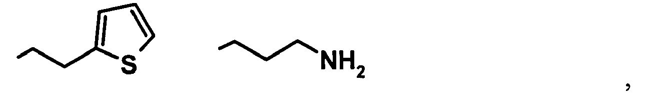 where the linking to the parent structure of the formula I, All or AIII in each case takes place via the bond to the left, which is not a methyl group,
in the sub-formula Ab
R^{2'}, R^{2"}, R^{2"'} each, independently of one another, denote H, methoxy, ethoxy, n-propoxy or i-propoxy,
in the sub-formula Ac
R^{2'}, R^{2"}, R^{2"'} each, independently of one another, denote H or methoxy,
in the sub-formula Ad
R^{2'}, R^{2"}, R^{2'"} each denote methoxy,
in the sub-formula Ae
R^{2'}, R^{2"}, R^{2'"} each, independently of one another, denote H, methoxy, ethoxy, n-propoxy or i-propoxy
and R¹ has the meaning indicated for the sub-formula Aa,
in the sub-formula Af
R^{2'}, R^{2"}, R^{2"'} each, independently of one another, denote H or methoxy
and R¹ has the meaning indicated for the sub-formula Aa,
in the sub-formula Ag
R^{2'}, R^{2"}, R^{2'"} each denote methoxy
and R¹ has the meaning indicated for the sub-formula Aa,
and pharmaceutically acceptable salts, solvates and stereoisomers thereof, including mixtures thereof in all ratios.

5. Compounds according to Claim 1 which conform to the formula BII 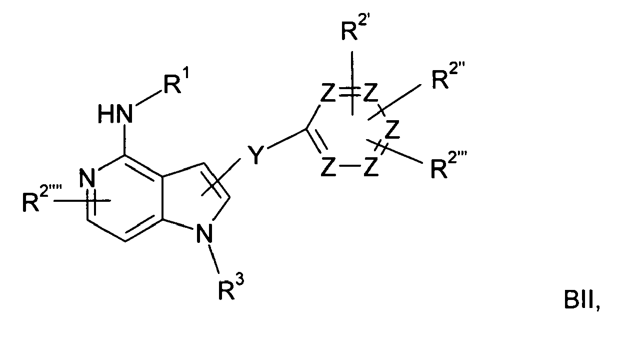 in which R¹, R², R³, Y and Z have the meaning indicated for the formula I according to Claim 1, and pharmaceutically acceptable salts, solvates and stereoisomers thereof, including mixtures thereof in all ratios.

6. Compounds according to Claim 1 or 5 which conform to the formula BIII 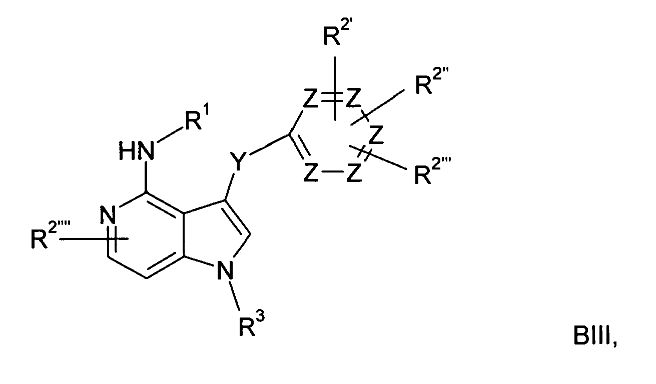 in which all radicals have the meaning indicated for the formula I according to Claim 1,
and pharmaceutically acceptable salts, solvates and stereoisomers thereof, including mixtures thereof in all ratios.

7. Compounds according to one or more of Claims 1, 5 and 6, in which the radicals not designated in greater detail have the meaning indicated for the formula I, but in which
in the sub-formula Ba
R¹ denotes phenyl, phenylmethyl, pyridyl, pyridylmethyl, pyridylethyl, pyrimidyl, piperazyl, quinolinyl, imidazoyl, imidazoylpropyl, pyrrolyl, pyrrolylethyl, each of which is unsubstituted or mono- or polysub- stituted by Hal, cyano, methyl or methoxy, and H
and R² and R³ have the meaning indicated for the formula I,
in the sub-formula Bb
R^{2'}, R^{2"}, R^{2"'} each, independently of one another, denote H, methoxy, ethoxy, n-propoxy, i-propoxy, phenylmethoxy or phenyl- ethoxy,
R^{2""} denotes H, Hal or NH₂
and R¹ and R³ have the meaning indicated for the formula I,
in the sub-formula Bc
one of the radicals R^{2'}, R^{2"}, R^{2"'} denotes methoxy or phenylmethoxy and the other two denote H
and R¹ and R³ have the meaning indicated for the formula I,
in the sub-formula Bd
R³ denotes 2-aminoethyl, 3-aminopropyl, 4-aminobutyl, 5-amino- pentyl, 3-aminomethylcyclobutyl, (isoindole-1,3-dion)-2-yl or 4-(tert- butyl carbamate)but-1-yl
and R¹ and R² have the meaning indicated for the formula I,
in the sub-formula Be
R^{2'}, R^{2"}, R^{2'"} each, independently of one another, denote H, methoxy, ethoxy, n-propoxy, i-propoxy, phenylmethoxy or phenylethoxy,
R^{2""} denotes H, Cl or NH₂,
R¹ denotes phenyl, phenylmethyl, pyridyl, pyridylmethyl, pyridyl- ethyl, pyrimidyl, piperazyl, quinolinyl, imidazoyl, imidazoyl- propyl, pyrrolyl, pyrrolylethyl, each of which is unsubstituted or mono- or polysubstituted by Hal, cyano, methyl or methoxy, and H and
R³ denotes 2-aminoethyl, 3-aminopropyl, 4-aminobutyl, 5-amino- pentyl, 3-aminomethylcyclobutyl, (isoindole-1,3-dion)-2-yl or 4-(tert-butyl carbamate)but-1-yl,
in the sub-formula Bf
one of the radicals R^{2'}, R^{2"}, R^{2"'} denotes methoxy or phenylmethoxy and the other two denote H,
R¹ denotes H, pyridyl, pyridylmethyl or (4-methoxyphenyl)methyl and
R³ denotes 2-aminoethyl, 3-aminopropyl, 4-aminobutyl, 5-amino- pentyl, 3-aminomethylcyclobutyl, (isoindole-1,3-dion)-2-yl or 4-(tert-butyl carbamate)but-1-yl,
in the sub-formula Bg
one of the radicals R^{2'}, R^{2"}, R^{2"'} denotes methoxy or phenylmethoxy and the other two denote H
R¹ denotes H, pyrid-2- or -3-yl, pyrid-2- or -3-ylmethyl or (4- methoxyphenyl)methyl and
R³ denotes 2-aminoethyl, 3-amino-n-propyl, 4-amino-n-butyl, 5-amino-n-pentyl, 3-aminomethylcyclobut-1-yl, (isoindole-1,3- dion)-2-yl or 4-(tert-butyl carbamate)but-1-yl
and pharmaceutically acceptable salts, solvates and stereoisomers thereof, including mixtures thereof in all ratios.

8. Process for the preparation of compounds of the formula I and physiologically acceptable salts, solvates and stereoisomers thereof, **characterised in that**
a compound of the formula (ix) 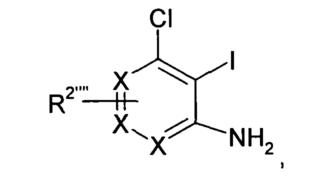 in which X has the meaning indicated for the formula I, is reacted with a compound of the formula (viii), 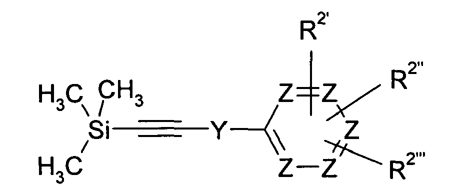 in which Z, Y and R² have the meanings indicated for the formula I, to give a compound of the formula (vii), 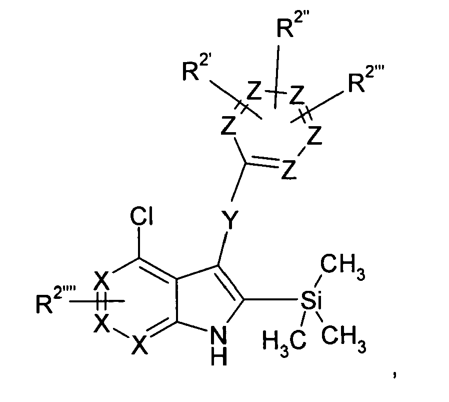 which is, if desired, reduced to give a corresponding 2,3-dihydroindole compound, which is, in a next step, converted into a compound of the formula (vi) 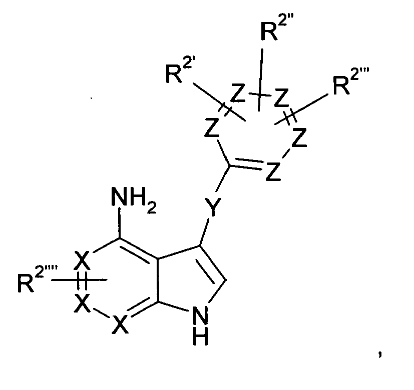 to which, if desired, a radical R¹ and/or a radical R³ are/is also attached to give a compound of the formula (iii) 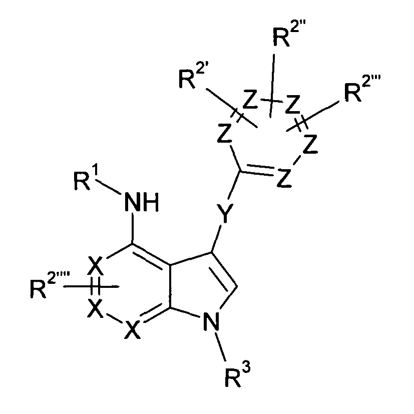 and, if desired, a base or acid of the formula (iii) is converted into one of its salts,
where, if the linking of the monocyclic aryl moiety to the bicyclic aryl moiety via its 2-position to give a compound of the formula (ii) 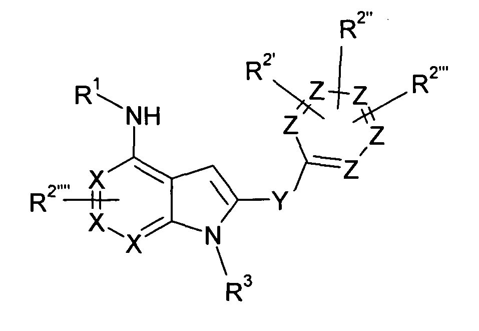 is desired, a compound of the formula (viii) without a silyl group is employed in the first step.

9. Compounds according to one or more of Claims 1 to 7 and/or physiologically acceptable salts, solvates and stereoisomers thereof, including mixtures thereof in all ratios, as medicaments.

10. Medicaments comprising at least one compound according to one or more of Claims 1 to 7 and/or physiologically acceptable salts, solvates and stereoisomers thereof, including mixtures thereof in all ratios, and optionally excipients and/or adjuvants.

11. Medicaments comprising at least one compound according to one or more of Claims 1 to 7 and/or physiologically acceptable salts, solvates and stereoisomers thereof, including mixtures thereof in all ratios, and at least one further medicament active ingredient.

12. Set (kit) consisting of separate packs of
a) an effective amount of a compound according to one or more of Claims 1 to 7 and/or physiologically acceptable salts, solvates and stereoisomers thereof, including mixtures thereof in all ratios, and
b) an effective amount of a further medicament active ingredient.

13. Compounds according to one or more of Claims 1 to 7 and physiologically acceptable salts, solvates and stereoisomers thereof, including mixtures thereof in all ratios, as activators or inhibitors of kinases, in particular tyrosine kinases.

14. Compounds according to one or more of Claims 1 to 7 and physiologically acceptable salts, solvates and stereoisomers thereof, including mixtures thereof in all ratios, as inhibitors of the receptor tyrosine kinase IGF-1 R.

15. Use of compounds according to one or more of Claims 1 to 7 and/or physiologically acceptable salts, solvates and stereoisomers thereof, including mixtures thereof in all ratios, for the preparation of a medicament for the prophylaxis or treatment of diseases in which inhibition of the receptor tyrosine kinase IGF-1 R results in an improvement in the clinical picture.

16. Use of compounds according to one or more of Claims 1 to 7 and/or physiologically acceptable salts, solvates and stereoisomers thereof, including mixtures thereof in all ratios, for the preparation of a medicament for the prophylaxis or treatment of cancer, tumour growth, tumour angiogenesis, arteriosclerosis, diabetic retinopathy and inflammatory diseases.

17. Use of compounds according to one or more of Claims 1 to 7 and/or physiologically acceptable salts, solvates and stereoisomers thereof, including mixtures thereof in all ratios, for the preparation of a medicament for the prophylaxis or treatment of breast cancer, prostate cancer, colorectal cancer, small-cell lung cancer, non-small-cell lung cancer, multiple myeloma and renal-cell carcinoma and endometrial carcinoma.

## Revendications

1. Composés de formule I, 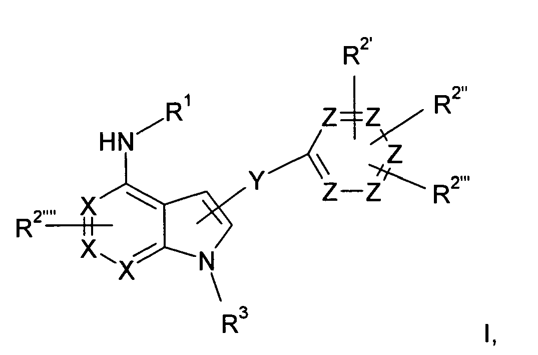 dans laquelle
R¹ désigne H, A, Ar, Ar-A ou A-Ar,
A désigne alkyle non ramifié, ramifié ou cyclique ayant 1-14 atomes de C, où un ou deux groupements CH₂ peuvent être remplacés par un atome de O ou de S et/ou par un groupe- ment NH, NA, CONH, NHCO ou -CH=CH- et/ou, en outre, 1-7 atomes de H peuvent être remplacés par Hal, et où un ou deux groupements CH₃ peuvent être remplacés par NH₂, NAH, NA₂, NHCOOA, NHCONHA, NHCONHAr ou CN,
Ar désigne un homo- ou hétérocycle aromatique mono- ou bicyclique ayant 1 à 4 atomes de N, de O et/ou de S et 5 à 10 atomes du squelette, pouvant être non substitué ou mono-, di- ou trisubstitué par oxygène carbonyle, Hal, A, OH, OA, NH₂, NHA, NA₂, NO₂, CN, OCN, SCN, COOH, COOA, CONH₂, CONHA, CONA₂, NHCOA, NHCONH₂, NHSO₂A CHO, COA, SO₂NH₂ et/ou S(O)gA,
Ar-A désigne arylalkyle,
A-Ar désigne alkylaryle,
Hal désigne F, Cl, Br ou I,
X désigne CH ou N, où un groupement X dans chaque com- posé de la formule I est N et deux groupements X sont CH,
Y désigne CH₂ ou une liaison saturée,
Z désigne CH ou N, où au plus deux groupements Z dans chaque composé de la formule I sont NH et de préférence un groupement Z, ou aucun, est NH,
R^{2'}, R^{2"}, R^{2"'}, R^{2""} désignent chacun, indépendamment les uns des autres, H, Hal, OH, CN, NH₂, alkyle non ramifié ou ramifié ayant 1-4, 5 ou 6 atomes de C, où un groupement CH₂ peut être rem- placé par un atome de O ou de S et/ou par un groupement NH, NA, CONH ou -CH=CH- et/ou, en outre, 1-4 atomes de H peuvent être remplacés par Hal, et où un groupement CH₃ peut être remplacé par NH₂, NAH, NA₂, CN ou Ar,
R³ désigne H, A ou Ar-A,
g désigne 0, 1 ou 2 et
-̅ -̅ -̅ -̅ -̅ désigne une liaison simple ou double,
la 3-(aminophényl)-1H-pyrrolo[3,2-c]pyridin-4-amine n'est pas incluse,
et les sels pharmaceutiquement acceptables, les solvats et les stéréoisomères de ceux-ci, y compris des mélanges de ceux-ci en toutes proportions.

2. Composés selon la revendication 1, répondant à la formule All 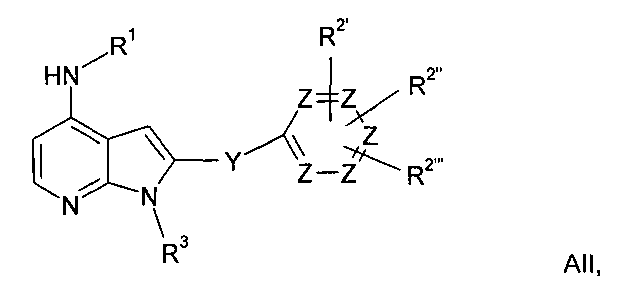 dans laquelle R¹, R², R³, Y et Z ont les significations indiquées pour la formule I selon la revendication 1, et les sels pharmaceutiquement acceptables, les solvats et les stéréoisomères de ceux-ci, y compris des mélanges de ceux-ci en toutes proportions.

3. Composés selon la revendication 1 ou 2, répondant à la formule Alll 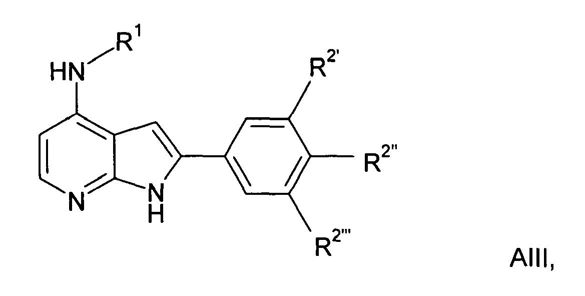 dans laquelle Y désigne une liaison, Z désigne CH, R³ désigne H, et R¹ et R² ont les significations indiquées pour la formule I selon la revendication 1,
et les sels pharmaceutiquement acceptables, les solvats et les stéréoisomères de ceux-ci, y compris des mélanges de ceux-ci en toutes proportions.

4. Composés selon l'une ou plusieurs parmi les revendications 1 à 3, dans lesquels les radicaux non désignés plus en détail ont les significations indiquées pour la formule I selon la revendication 1, mais où
dans la sous-formule Aa
R¹ désigne phényle, phénylméthyle, pyridyle, pyridylméthyle, pyridyl- éthyle, pyrimidyle, pipérazinyle, quinoléinyle, imidazolyle, imida- zoylpropyle, pyrrolyle, pyrrolyléthyle, chacun d'entre eux étant non substitué ou mono- ou polysubstitué par Hal, cyano, méthyle ou méthoxy, de plus H, N,N'-diméthylaminopropyle ou cyanobutyle ou l'un parmi les radicaux suivants: 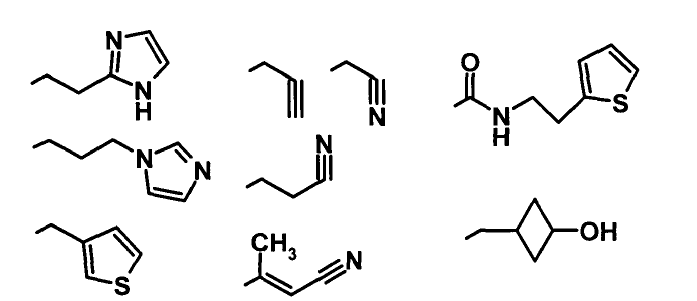 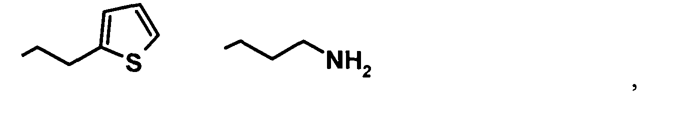 où la liaison vers la structure parente de formule I, AII ou AIII dans chaque cas se fait par la liaison à gauche, qui n'est pas un groupe- ment méthyle,
dans la sous-formule Ab
R^{2'}, R^{2"}, R^{2'"} désignent chacun, indépendamment les uns des autres, H, méthoxy, éthoxy, n-propoxy ou i-propoxy,
dans la sous-formule Ac
R^{2'}, R^{2"}, R^{2"'} désignent chacun, indépendamment les uns des autres, H ou méthoxy,
dans la sous-formule Ad
R^{2'}, R^{2"}, R^{2'"} désignent chacun méthoxy,
dans la sous-formule Ae
R^{2'}, R^{2"}, R^{2"'} désignent chacun, indépendamment les uns des autres, H, méthoxy, éthoxy, n-propoxy ou i-propoxy
et R¹ a la signification indiquée pour la sous-formule Aa,
dans la sous-formule Af
R^{2'}, R^{2"}, R^{2'"} désignent chacun, indépendamment les uns des autres, H ou méthoxy
et R¹ a la signification indiquée pour la sous-formule Aa, dans la sous-formule Ag
R^{2'}, R^{2''}, R^{2'''} désignent chacun méthoxy
et R¹ a la signification indiquée pour la sous-formule Aa,
et les sels pharmaceutiquement acceptables, les solvats et les stéréoisomères de ceux-ci, y compris des mélanges de ceux-ci en toutes proportions.

5. Composés selon la revendication 1, répondant à la formule BII 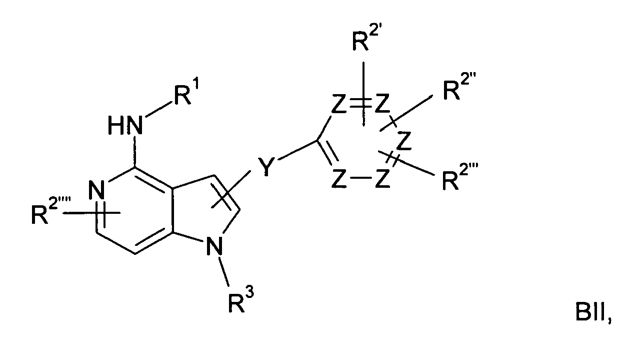 dans laquelle R¹, R², R³, Y et Z ont les significations indiquées pour la formule I selon la revendication 1, et les sels pharmaceutiquement acceptables, les solvats et les stéréoisomères de ceux-ci, y compris des mélanges de ceux-ci en toutes proportions.

6. Composés selon la revendication 1 ou 5, répondant à la formule BIII 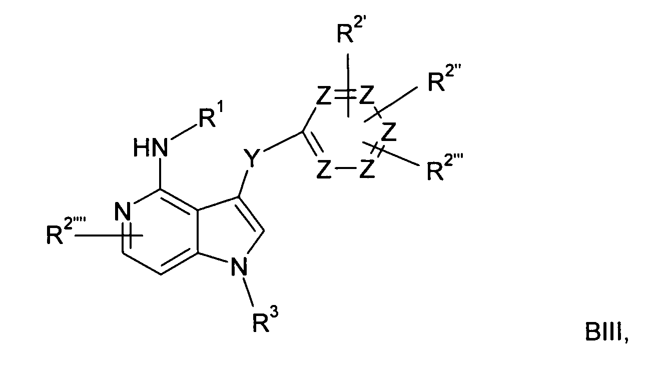 dans laquelle tous les radicaux ont les significations indiquées pour la formule 1 selon la revendication 1,
et les sels pharmaceutiquement acceptables, les solvats et les stéréoisomères de ceux-ci, y compris des mélanges de ceux-ci en toutes proportions.

7. Composés selon l'une ou plusieurs parmi les revendications 1, 5 et 6, dans lesquels les radicaux non désignés plus en détail ont les significations indiquées pour la formule I, mais où
dans la sous-formule Ba
R¹ désigne phényle, phénylméthyle, pyridyle, pyridylméthyle, pyridyl- éthyle, pyrimidyle, pipérazinyle, quinoléinyle, imidazolyle, imidazo- lylpropyle, pyrrolyle, pyrrolyléthyle, chacun d'entre eux étant non substitué ou mono- ou polysubstitué par Hal, cyano, méthyle ou méthoxy, et H
et R² et R³ ont les significations indiquées pour la formule I,
dans la sous-formule Bb
R^{2'} , R^{2"}, R^{2"'} désignent chacun, indépendamment les uns des autres, H, méthoxy, éthoxy, n-propoxy, i-propoxy, phénylméthoxy ou phényléthoxy,
R^{2""} désigne H, Hal ou NH₂
et R¹ et R³ ont les significations indiquées pour la formule I,
dans la sous-formule Bc
l'un parmi les radicaux R^{2'}, R^{2"}, R^{2"'} désigne méthoxy ou phénylméthoxy et les deux autres désignent H
et R¹ et R³ ont les significations indiquées pour la formule I,
dans la sous-formule Bd
R³ désigne 2-aminoéthyle, 3-aminopropyle, 4-aminobutyle, 5-amino- pentyle, 3-aminométhylcyclobutyle, (isoindole-1,3-dion)-2-yle ou 4-(carbamate de tertio-butyl)but-1-yle
et R¹ et R² ont les significations indiquées pour la formule I, dans la sous-formule Be
R^{2'}, R^{2"}, R^{2'"} désignent chacun, indépendamment les uns des autres, H, méthoxy, éthoxy, n-propoxy, i-propoxy, phénylméthoxy ou phényléthoxy,
R^{2""} désigne H, Cl ou NH₂,
R¹ désigne phényle, phénylméthyle, pyridyle, pyridylméthyle, pyridyléthyle, pyrimidyle, pipérazinyle, quinoléinyle, imidazo- lyle, imidazolylpropyle, pyrrolyle, pyrrolyléthyle, chacun d'entre eux étant non substitué ou mono- ou polysubstitué par Hal, cyano, méthyle ou méthoxy, et H et
R³ désigne 2-aminoéthyle, 3-aminopropyle, 4-aminobutyle, 5-aminopentyle, 3-aminométhylcyclobutyle, (isoindole-1,3- dion)-2-yle ou 4-(carbamate de tertio-butyl)but-1-yle,
dans la sous-formule Bf
l'un parmi les radicaux R^{2'}, R^{2"}, R^{2"'} désigne méthoxy ou phénylméthoxy et les deux autres désignent H,
R¹ désigne H, pyridyle, pyridylméthyle ou (4-méthoxyphényl)- méthyle et
R³ désigne 2-aminoéthyle, 3-aminopropyle, 4-aminobutyle, 5-aminopentyle, 3-aminométhylcyclobutyle, (isoindole-1,3- dion)-2-yle ou 4-(carbamate de tertio-butyl)but-1-yle,
dans la sous-formule Bg
l'un parmi les radicaux R^{2'}, R^{2"}, R^{2"'} désigne méthoxy ou phénylméthoxy et les deux autres désignent H
R¹ désigne H, pyrid-2- ou -3-yle, pyrid-2- ou -3-ylméthyle ou (4- méthoxyphényl)méthyle et
R³ désigne 2-aminoéthyle, 3-amino-n-propyle, 4-amino-n-butyle, 5-amino-n-pentyle, 3-aminométhylcyclobut-1-yle, (isoindole- 1,3-dion)-2-yle ou 4-(carbamate de tertio-butyl)but-1-yle
et les sels pharmaceutiquement acceptables, les solvats et les stéréoisomères de ceux-ci, y compris des mélanges de ceux-ci en toutes proportions.

8. Procédé de préparation de composés de formule I et de sels physiologiquement acceptables, de solvats et de stéréoisomères de ceux-ci,
**caractérisé en ce que**
un composé de formule (ix) 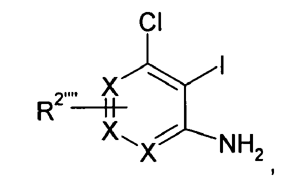 dans laquelle X a la signification indiquée pour la formule I, est réagi avec un composé de formule (viii), 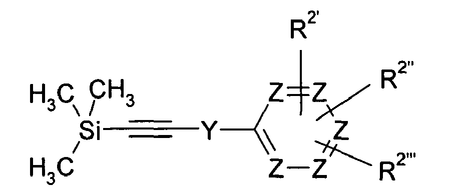 dans laquelle Z, Y et R² ont les significations indiquées pour la formule I, pour donner un composé de formule (vii), 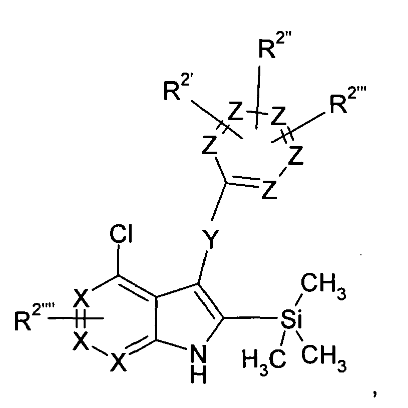 lequel est, si on le souhaite, réduit pour donner un composé de 2,3-dihydroindole correspondant, lequel est, dans une étape suivante, converti en un composé de formule (vi) 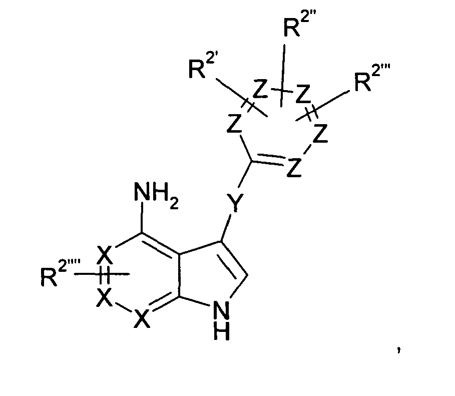 auquel, si on le souhaite, un radical R¹ et/ou un radical R³ est/sont également lié(s) pour donner un composé de formule (iii) 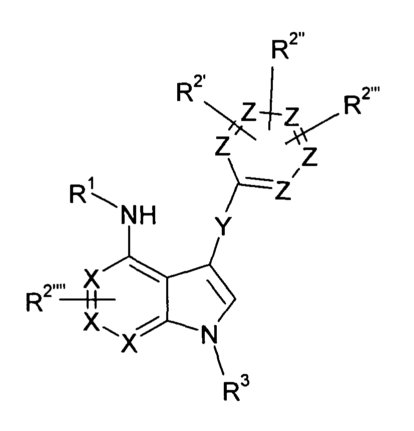 et, si on le souhaite, une base ou un acide de formule (iii) est converti(e) en l'un de ses sels,
où, si la liaison du motif aryle monocyclique au motif aryle bicyclique via sa position 2 pour donner un composé de formule (ii) 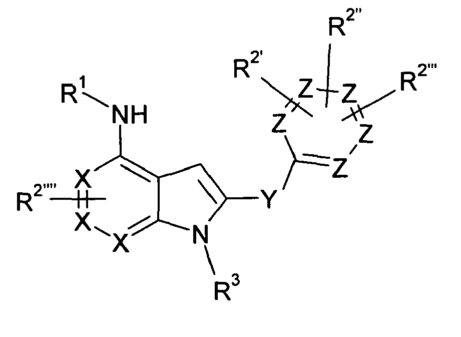 est désirée, un composé de formule (viii) dépourvu de groupement silyle est employé dans la première étape.

9. Composés selon l'une ou plusieurs parmi les revendications 1 à 7 et/ou des sels physiologiquement acceptables, des solvats et des stéréoisomères de ceux-ci, y compris des mélanges de ceux-ci en toutes proportions, comme médicaments.

10. Médicaments comprenant au moins un composé selon l'une ou plusieurs parmi les revendications 1 à 7 et/ou des sels physiologiquement acceptables, des solvats et des stéréoisomères de celui-ci, y compris des mélanges de ceux-ci en toutes proportions, et éventuellement des excipients et/ou adjuvants.

11. Médicaments comprenant au moins un composé selon l'une ou plusieurs parmi les revendications 1 à 7 et/ou des sels physiologiquement acceptables, des solvats et des stéréoisomères de celui-ci, y compris des mélanges de ceux-ci en toutes proportions, et au moins un autre ingrédient actif médicamenteux.

12. Ensemble (kit) constitué de conditionnements séparés
a) d'une quantité efficace d'un composé selon l'une ou plusieurs parmi les revendications 1 à 7 et/ou de sels physiologiquement acceptables, de solvats et de stéréoisomères de celui-ci, y compris des mélanges de ceux-ci en toutes proportions, et
b) d'une quantité efficace d'un autre ingrédient actif médicamenteux.

13. Composés selon l'une ou plusieurs parmi les revendications 1 à 7 et des sels physiologiquement acceptables, des solvats et des stéréoisomères de ceux-ci, y compris des mélanges de ceux-ci en toutes proportions, comme activateurs ou inhibiteurs de kinases, en particulier de tyrosine kinases.

14. Composés selon l'une ou plusieurs parmi les revendications 1 à 7 et des sels physiologiquement acceptables, des solvats et des stéréoisomères de ceux-ci, y compris des mélanges de ceux-ci en toutes proportions, comme inhibiteurs du récepteur à activité tyrosine kinase IGF-1 R.

15. Utilisation de composés selon l'une ou plusieurs parmi les revendications 1 à 7 et/ou de sels physiologiquement acceptables, de solvats et de stéréoisomères de ceux-ci, y compris des mélanges de ceux-ci en toutes proportions, pour la préparation d'un médicament destiné à la prophylaxie ou au traitement de maladies dans lesquelles l'inhibition du récepteur à activité tyrosine kinase IGF-1 R entraîne une amélioration du tableau clinique.

16. Utilisation de composés selon l'une ou plusieurs parmi les revendications 1 à 7 et/ou de sels physiologiquement acceptables, de solvats et de stéréoisomères de ceux-ci, y compris des mélanges de ceux-ci en toutes proportions, pour la préparation d'un médicament destiné à la prophylaxie ou au traitement de cancers, de la croissance tumorale, de l'angiogenèse de tumeurs, de l'artériosclérose, de la rétinopathie diabétique et de maladies inflammatoires.

17. Utilisation de composés selon l'une ou plusieurs parmi les revendications 1 à 7 et/ou de sels physiologiquement acceptables, de solvats et de stéréoisomères de ceux-ci, y compris des mélanges de ceux-ci en toutes proportions, pour la préparation d'un médicament destiné à la prophylaxie ou au traitement du cancer du sein, du cancer de la prostate, du cancer colorectal, du cancer du poumon à petites cellules, du cancer du poumon non à petites cellules, du myélome multiple et de l'hypernéphrome et du carcinome de l'endomètre.
